# EUROPEAN PATENT SPECIFICATION

(11) **EP 0 642 502 B1**
(45) Date of publication and mention of the grant of the patent: **12.07.2000**
(21) Application number: 93911196.9
(22) Date of filing: 14.05.1993
(51) Int. Cl.: C07D 233/86, C07D 233/72, C07D 233/70, C07D 235/02, C07D 409/04, C07D 495/10, C07D 491/107, C07D 405/04, C07D 401/04, A01N 43/50, A01N 43/52

(54) **FUNGICIDAL IMIDAZOLINONES**
FUNGIZIDE IMIDAZOLINONE
IMIDAZOLINONES FONGICIDES

(30) Priority: 22.05.1992 US 887528
(43) Date of publication of application: 15.03.1995
(60) Divisional application: 00106172.0
(73) Proprietor: E.I. DU PONT DE NEMOURS AND COMPANY, Wilmington Delaware 19898 (US)
(72) Inventor: SUN, King-Mo, Hockessin, DE 19707 (US)
(74) Representative: Woodman, Derek
(86) International application number: US9304396
(87) International publication number: WO9324467

(56) References cited:
- EP-A- 0 551 048
- WO-A-88/07040
- WO-A-90/12791

## Description

This invention relates to particular imidazolinone compounds useful as fungicides, agriculturally suitable compositions containing such compounds, and methods of use of such compounds or compositions as fungicides in crop plants.

WO90/12791 is drawn to the use of fungicidal compounds of Formula i 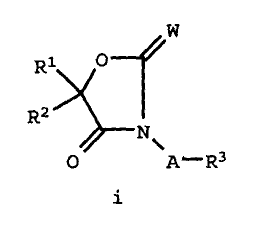 wherein:
A is O or NR⁴; and
W is O or S.

WO90/12791 also relates to processes for the preparation of compounds of Formula i and to certain novel compounds.

The compounds of WO90/12791 are distinct from those of the present invention in that oxygen is incorporated into the central heterocyclic ring of compounds of Formula i and the bonds forming this five-membered ring are all single bonds.

EP-A-0551048 forms part of the state of the art for novelty-only purposes (Article 54(3) EPC), and discloses racemic fungicidal 2-imidazoline-5-one and 2-imidazoline-5-thione derivatives.

### SUMMARY OF THE INVENTION

This invention comprises compounds of Formula I including all geometric and stereoisomers thereof, agricultural compositions containing one or more such compounds, and methods of use of such compounds or compositions as fungicides.

The compounds of the present invention have the following structure: 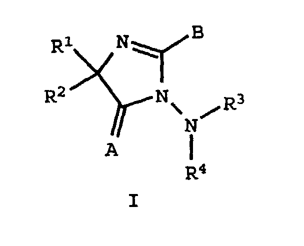 wherein:
- A: is O or NH;
- B: is halogen; cyano; R²¹; OR²⁹; NR⁴⁹R⁶³; N=CR⁴⁵R⁴⁶; SR⁴⁷; or S(O)₂R⁴⁸;
- R¹: is C₁-C₄ alkyl; C₁-C₄ haloalkyl; or vinyl;
- R²: is C₂-C₂₀ alkyl; C₂-C₂₀ alkoxyalkyl; C₃-C₈ alkyl substituted with phenoxy or phenylthio each optionally substituted with R³⁰; C₂-C₂₀ alkenyl; C₅-C₇ cycloalkyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷;
provided that when R² is phenyl and R⁵ is other than F, then R⁵ is attached to the para-position relative to the imidazolinone ring;
- R³: is phenyl optionally substituted with R¹⁰;
- R⁴: is H or methyl;
- R⁵: is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁴; phenylmethyl, phenoxymethyl, phenethyl, or styryl each optionally substituted with R²⁴ on the phenyl ring; phenoxy optionally substituted with R²⁷; benzyloxy optionally substituted with R³⁰ on the phenyl ring; -OC(=O)NHR²⁸; -C(=O)OR²⁸; or -OC(=O)R²⁸;
- R⁷, R²⁴ and R²⁵: are independently 1-2 halogen; nitro; C₁-C₄ alkyl; trifluoromethyl; methylthio; or C₁-C₄ alkoxy;
- R¹⁰: is 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
- R²¹: is C₁-C₄ alkyl optionally substituted with R⁵¹; C₂-C₄ alkenyl, C₂-C₈ alkynyl, or cyclopropyl each optionally substituted with 1-3 halogen; C(=N-V-R⁵³)H; C(=N-V-R⁵³) (C₁-C₄ alkyl); C(=O)OR⁵³; C(=O)SR⁵³; C(=S)SR⁵³; or C(=O)NR⁵³R⁵⁶;
- V: is O; NR⁵⁵; or a direct bond;
- R²⁷: is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; hydroxycarbonyl; C₂-C₄ alkoxycarbonyl; or phenoxy optionally substituted with R²⁴;
- R²⁸: is C₁-C₈ alkyl; or phenyl or pyridyl each optionally substituted with R³⁰;
- R²⁹: is C₁-C₄ alkyl optionally substituted with R⁴⁴; C₃-C₆ alkenyl, C₃-C₆ alkynyl, or cyclopropyl, each optionally substituted with 1-3 halogen; C(=O)R⁵²; C(=NR⁵⁵)R⁵²; C(=O)OR⁵³; C(=O)NR⁵³R⁵⁶; N=CR⁶⁸R⁶⁷; or SO₂R⁵²;
- R³⁰: is 1-2 substituents selected from the group consisting of halogen, nitro, cyano, C₁-C₄ alkyl, trifluoromethyl, C₁-C₄ alkoxy and trifluoromethoxy; or phenoxy optionally substituted with R²⁶;
- R⁴⁴: is 1-3 halogen; C₁-C₄ alkoxy; or C₁-C₄ haloalkoxy;
- R⁴⁵: is H; or C₁-C₄ alkyl;
- R⁴⁶: is H; C₁-C₄ alkyl; OR⁶⁵; or SR⁶⁵;
- R⁴⁷: is C₁-C₄ alkyl; C₃-C₄ alkenyl; C(=O)R⁵²; or C(=O)OR⁵³;
- R⁴⁸: is C₁-C₄ alkyl;
- R⁴⁹: is H; C₁-C₄ alkyl; C₃-C₄ alkenyl; or cyclopropyl;
- R⁵¹: is 1-3 halogen or C₂-C₃ haloalkoxy;
- R⁵² and R⁵³: are each independently H; C₁-C₄ alkyl; or C₃-C₄ alkenyl;
- R⁵⁵ and R⁵⁶: are each independently H or C₁-C₄ alkyl;
- R⁶³: is C₁-C₄ alkyl;
- R⁶⁵: is C₁-C₄ alkyl; or C₃-C₄ alkenyl;
- R⁶⁷: is H or C₁-C₄ alkyl; and
- R⁶⁸: is H; C₁-C₄ alkyl; C₁-C₄ haloalkyl; or alkenyl;
provided that the total number of carbons in R² is less than or equal to 20; and
provided that when R³ is a phenyl or heterocyclic ring disubstituted with two alkyl or alkoxy groups, or one alkyl and one alkoxy group, then at least one of the alkyl and alkoxy groups is methyl or methoxy.

### DETAILED DESCRIPTION OF THE INVENTION

In the above recitations, the term "alkyl", used either alone or in compound words such as "alkylthio" or "haloalkyl" denotes straight-chain or branched alkyl; e.g., methyl, ethyl, *n*-propyl, *i*-propyl, or the different butyl, pentyl or hexyl isomers.

"Alkenyl" denotes straight-chain or branched alkenes; e.g., 1-propenyl, 2-propenyl, 3-propenyl and the different butenyl, pentenyl and hexenyl isomers. "Alkenyl" also denotes polyenes such as 1,3-hexadiene and 2,4,6-heptatriene.

"Alkenyloxy" denotes straight-chain or branched alkenyloxy moieties. Examples of alkenyloxy include H₂C=CHCH₂O, (CH₃)₂C=CHCH₂O, (CH₃)CH=CHCH₂O, (CH₃)CH=C(CH₃)CH₂O and CH₂=CHCH₂CH₂O.

"Alkynyl" denotes straight-chain or branched alkynes; e.g., ethynyl, 1-propynyl, 3-propynyl and the different butynyl, pentynyl and hexynyl isomers. "Alkynyl" can also denote moieties comprised of multiple triple bonds; e.g., 2,7-octadiyne and 2,5,8-decatriyne.

"Alkynyloxy" denotes straight-chain or branched alkynyloxy moieties. Examples include HC≡CCH₂O, CH₃C≡CCH₂O and CH₃C≡CCH₂CH₂O.

"Alkylthio" denotes branched or straight-chain alkylthio moieties; e.g., methylthio, ethylthio, and the different propylthio, butylthio, pentylthio and hexylthio isomers.

Examples of "alkylsulfonyl" include CH₃SO₂, CH₃CH₂SO₂, CH₃CH₂CH₂SO₂, (CH₃)₂CHSO₂ and the different butylsulfonyl, pentylsulfonyl and hexylsulfonyl isomers.

"Alkylsulfinyl" denotes both enantiomers of an alkylsulfinyl group. For example, CH₃SO, CH₃CH₂SO, CH₃CH₂CH₂SO, (CH₃)₂CHSO and the different butylsulfinyl, pentylsulfinyl and hexylsufinyl isomers.

"Alkoxy" denotes, for example, methoxy, ethoxy, *n*-propyloxy, isopropyloxy and the different butoxy, pentoxy and hexyloxy isomers.

Examples of "alkoxyalkoxyalkoxy" include CH₃OCH₂CH₂OCH₂O, CH₃CH₂OCH₂CH₂OCH₂O, and (CH₃)₂CHOCH₂CH₂OCH₂O.

"Cycloalkyl" denotes, for example, cyclopropyl, cyclobutyl, cyclopentyl, and cyclohexyl. The term "cycloalkyloxy" denotes the same groups linked through an oxygen atom such as cyclopentyloxy and cyclohexyloxy. "Cycloalkenyl" denotes groups such as cyclopentenyl and cyclohexenyl.

Examples of "cycloalkylalkyl" include cyclopropylmethyl, cyclohexylethyl, and other cycloalkyl moieties bonded to straight-chain or branched alkyl groups. "Alkylcycloalkyl" denotes alkyl substitution on a cycloalkyl moiety. Examples include 4-methylcyclohexyl and 3-isopropylcyclopentyl.

The term "halogen", either alone or in compound words such as "haloalkyl", denotes fluorine, chlorine, bromine or iodine. Further, when used in compound words such as "haloalkyl", said alkyl may be partially or fully substituted with halogen atoms which may be the same or different. Examples of "haloalkyl" include F₃C, ClCH₂, CF₃CH₂ and CF₃CF₂. Examples of "haloalkenyl" include (Cl)₂C=CHCH₂ and CF₃CH₂CH=CHCH₂. Examples of "haloalkynyl" include HC≡CCHCl, CF₃C≡C, CCl₃C≡C and FCH₂C≡CCH₂. Examples of "haloalkoxy" include CF₃O, CCl₃CH₂O, CF₂HCH₂CH₂O and CF₃CH₂O. Examples of "haloalkylthio" include CCl₃S, CF₃S, CCl₃CH₂S and CH₂ClCH₂CH₂S. Examples of "haloalkylsulfonyl" include CF₃SO₂, CCl₃SO₂, CF₃CH₂SO₂ and CF₃CF₂SO₂. Examples of "haloalkoxyalkoxy" include CF₃OCH₂O, ClCH₂CH₂OCH₂CH₂O, Cl₃CH₂OCH₂O as well as branched alkyl derivatives.

The total number of carbon atoms in a substituent group is indicated by the "Cᵢ-Cⱼ" prefix where i and j are numbers from 1 to 20. For example, C₁-C₃ alkylsulfonyl designates methylsulfonyl through propylsulfonyl; C₂ alkoxyalkoxy designates CH₃OCH₂O; C₃ alkoxyalkoxy designates, for example, CH₃OCH₂CH₂O or CH₃CH₂OCH₂O; and C₄ alkoxyalkoxy designates the various isomers of an alkoxy group substituted with a second alkoxy group containing a total of 4 carbon atoms, examples including CH₃CH₂CH₂OCH₂O, and CH₃CH₂OCH₂CH₂O. Examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂. Examples of "alkoxycarbonyl" include CH₃OC(=O), CH₃CH₂OC(=O), CH₃CH₂CH₂OC(=O), (CH₃)₂CHOC(=O) and the different butoxy-, pentoxy- or hexyloxycarbonyl isomers.

In the above recitations, when a compound of Formula I is comprised of one or more pyridyl or pyrimidinyl rings, all bonds to these heterocycles are made through the carbon atom(s) of the moieties. When a substituent for a compound of Formula I is defined to include 1-2 halogen or 1-3 halogen, this denotes the occurrence of the same or different halogens at that substituent position one, two, or three times.

When R¹ and R² of the compounds of Formula I are different, then the compounds of Formula I possess a chiral center. This invention, therefore, comprises racemic mixtures as well as pure enantiomers. Compounds of Formula I can also exist as (E) - or (Z)-isomers, or as a mixture of (E)- and (Z)-isomers when compounds of Formula I contain a C=C bond or a C=N bond. This invention, therefore, also comprises mixtures of geometric isomers as well as the individual isomers.

Preferred compounds of Formula I (denoted as Preferred 1) are the compounds wherein:
- B: is halogen; cyano; C₁-C₄ alkyl, or C₁-C₄ alkoxy each optionally substituted with halogen; C₁-C₄ alkylthio; N=CR⁴⁵R⁴⁶; NR⁴⁹R⁶³; or S(O)₂(C₁-C₄ alkyl);
- R¹: is methyl or halomethyl;
- R²: is C₂-C₁₂ alkyl; C₃-C₈ alkyl substituted with phenoxy optionally substituted with R³⁰; phenyl optionally substituted with R⁵ and R⁷ thienyl optionally substituted with R⁷; or pyridyl optionally substituted with R⁵ and R⁷;
- R³: is phenyl optionally substituted with F; Cl; or methyl;
- R⁴: is H;
- R⁵: is halogen; nitro; C₁-C₆ alkyl; C₁-C₃ haloalkyl; methylthio; C₁-C₆ alkoxy; C₁-C₂ haloalkoxy; C₅-C₆ cycloalkyloxy; phenoxy optionally substituted with R²⁷; phenylthio substituted with R²⁴; phenoxymethyl optionally substituted with R²⁴ on the phenyl ring; benzyloxy optionally substituted with R³⁰ on the phenyl ring; or -OC(=O)R²⁸;
- R⁷ and R²⁴: are independently F; C₁-C₂ alkyl; methylthio; or C₁-C₂ alkoxy;
- R²⁷: is 1-2 halogen; cyano; C₁-C₄ alkyl; trifluoromethyl; C₁-C₄ alkoxy; C₁-C₄ haloalkoxy; C₁-C₄ alkylthio; C₅-C₆ cycloalkyloxy; or allyl;
- R³⁰: is 1-2 halogen; cyano; C₁-C₄ alkyl; trifluoromethyl; C₁-C₄ alkoxy; or trifluoromethoxy;
- R⁴⁶: is H or C₁-C₄ alkyl;
- R⁴⁹ and R⁶³: are each independently C₁-C₂ alkyl.

Especially preferred compounds of Formula I (denoted as Preferred 2) are the compounds of Preferred 1 wherein:
- B: is F; Cl; cyano; C₁-C₂ alkyl; C₁-C₂ alkylthio; C₁-C₂ alkoxy; N=CR⁴⁵R⁴⁶; NMe₂; or S(O)₂(C₁-C₂ alkyl);
- R¹: is methyl;
- R²: is C₂-C₁₂ alkyl; phenyl optionally substituted with R⁵ and R⁷; or thienyl optionally substituted with R⁵ and R⁷; and
- R⁵: is F; Cl; Br; C₁-C₆ alkyl; trifluoromethyl; C₁-C₆ alkoxy; trifluoromethoxy; 2,2,2-trifluoroethoxy; C₅-C₆ cycloalkyloxy; methylthio; phenoxy optionally substituted with R²⁷; phenylthio optionally substituted with R²⁴; benzyloxy optionally substituted with R³⁰ on the phenyl ring; or -OC(=O)R²⁸.

Specifically preferred compounds of Formula I are the compounds of Preferred 2 which are:
3,5-dihydro-2-methoxy-5-methyl-5-phenyl-3-(phenylamino)-4H-imidazol-4-one; and
3,5-dihydro-5-methyl-2-(methylthio)-5-phenyl-3-(phenylamino)-4H-imidazol-4-one.

It is recognized that some reagents and reaction conditions described below for preparing compounds of Formula I may not be compatible with some functionalities claimed for R¹, R², R³, R⁴, A and B. In these cases, the incorporation of protection/deprotection sequences into the synthesis may be necessary in order to obtain the desired products. The cases in which protecting groups are necessary, and which protecting group to use, will be apparent to one skilled in chemical synthesis.

In the following description of the preparation of compounds of Formula I, compounds denoted as Formula Ia through Formula Ix are various subsets of the compounds of Formula I, and all substituents for Formula Ia through Ix are as defined above for Formula I.

The compounds of Formula I can be prepared as described below in the following Schemes. The 4(H)-imidazol-4-ones of Formula Ia can be prepared by one or all of the methods illustrated in Scheme I.

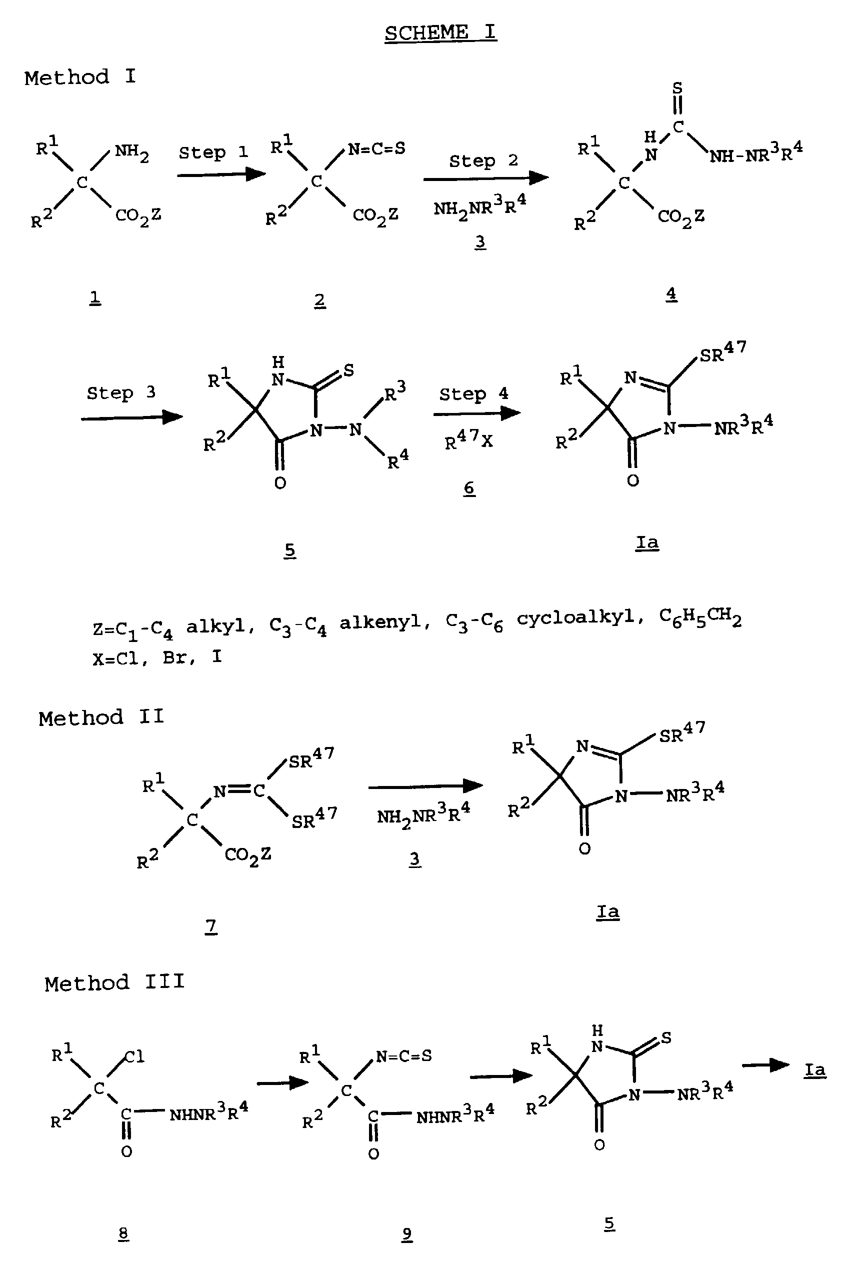

The esters of α-amino-acids of Formula 1 or their salts are known in the literature and can be prepared by literature methods [J. P. Greenstein, M. Winitz, "Chemistry of the Amino Acids" (S. Patai, Ed.), p 697, John Wiley and Sons, Ltd., London (1961)]. The group Z in compounds of Formula 1 can be C₁-C₄ alkyl; C₃-C₄ alkenyl; C₃-C₆ cycloalkyl; or C₆H₅CH₂. Preferred for ease of synthesis and lower expense are Z=C₁-C₄ alkyl. The preparation of the isothiocyanates of Formula 2 from the α-amino esters or their salts of Formula 1 (Step 1) can also be accomplished by literature methods (H. A. Staab and G. Walther, *Liebigs Ann. Chem.*, **1962**, *657*, 98; M. L. Moore, F. S. Crossley, *Org. Synth.*, **1941**, *21*, 81).

Compounds of Formula 4 can be prepared by treatment of isothiocyanates of Formula 2 with hydrazines of Formula 3 (Step 2). The preparation of substituted hydrazines of Formula 3 can be accomplished by literature methods (J. Timerblake, J. Stowell; "The Chemistry of the Hydrazo, Azo and Azoxy Groups" (S. Patai, Ed.) p 69, John Wiley and Sons, Ltd., London (1975); J. P. Demers, D. J. Klaubert, *Tetrahedron Lett.*, **1987**, 4933). To prepare compounds of Formula 4, the compounds of Formula 2 are dissolved in an inert solvent such as 1-chlorobutane or dichloromethane, and a hydrazine of Formula 3 is added, at a temperature from -50° to 50°C. When the reaction is complete, the resulting mixture is poured into a water-immiscible solvent and washed successively with dilute aqueous mineral acid, water, and brine. The organic phase of this mixture is separated, dried, and the solvent is evaporated to provide the products of Formula 4.

As shown in Step 3, the 2-thioxo-4-imidazolidinone compounds of Formula 5 can be prepared by dissolving compounds of Formula 4 in an inert solvent, such as benzene or toluene, and heating at a temperature from 50° to 200°C. When the reaction is complete, the solvent is evaporated and the resulting mixture is purified to yield products of Formula 5.

In some cases, the isolation of compounds of Formula 4 is unnecessary. For example, compounds of Formula 4 can be converted *in situ* to compounds of Formula 5 by warming the reaction mixture to 50°-200°C. When the reaction is complete, the solvent is removed to provide compounds of Formula 5.

The 4(H)-imidazol-4-ones of Formula Ia can be prepared by dissolving compounds of Formula 5 in an inert solvent, such as chloroform or tetrahydrofuran, and treating the solution with an electrophilic substrate R⁴⁷X wherein X is a leaving group such as a chlorine, bromine, or iodine; followed by a base such as 1,8-diaza-bicyclo[5.4.0]-undec-7-ene, at a temperature from 0° to 100°C (Step 4). When the reaction is complete, the solvent is evaporated and the resulting mixture is purified to yield products of Formula Ia.

An alternative process for preparing compounds of Formula Ia is illustrated in Method II, Scheme I. The α-bis(thio)methyleneamino acid esters of Formula 7 can be prepared by literature methods (D. Hoppe, *Angew. Chem.*, *Int. Ed. Eng.*, **1975**, *14*, 426). Compounds of Formula Ia can be prepared by dissolving compounds of Formula 7 in a protic acid, such as acetic acid, with or without an inert diluent, such as dichloromethane, in the presence of a hydrazine of Formula 3, at a temperature from 20° to 100°C. When the reaction is complete, the reaction mixture is poured into water and extracted with a water-immiscible organic solvent such as ethyl acetate. The combined organic extracts are washed first with aqueous base, such as aqueous sodium bicarbonate, and then with water. The organic layer is then dried and evaporated to yield compounds of Formula Ia.

Another route to synthesize compounds of Formula Ia is illustrated in Method III, Scheme I. This method is described in the literature for when R¹ is H and neither R³ nor R⁴ are H (H. Boehme, F. Martin, J. Strahl; *Arch. Pharm.* **1980**, *313*, 10).

As shown in Step 1, a α-chlorohydrazide, prepared by literature methods (H. Böhme, F. Martin, *Acad. Pharm.*, **1974**, *307*, 277), is treated with a salt of a thiocyanate, such as potassium thiocyanate, in an inert solvent, such as acetonitrile at a temperature from 0° to 50°C. When the reaction is complete, the reaction mixture is poured into water and extracted with a water-immiscible solvent such as chloroform. The organic extracts are combined and washed with water. The solvent is removed to give a residue which is redissolved and refluxed in an inert organic solvent such as acetonitrile. When the reaction is complete, the solvent is removed and the resulting mixture is purified to give a compound of Formula 5. The conversion of compounds of Formula 5 to compounds of Formula Ia is described in Step 4, Method I.

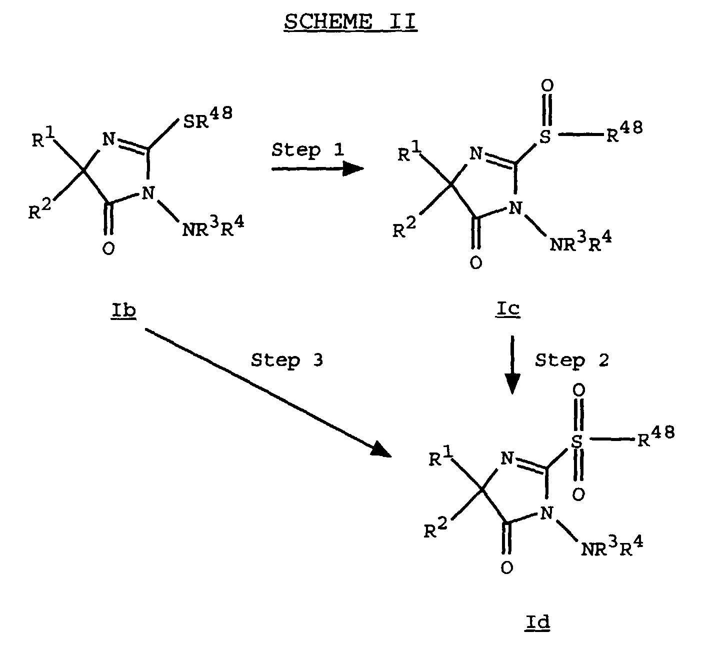

The sulfoxides and sulfones of Formula Ic and Id, respectively, can be prepared by the methods illustrated in Scheme II. The oxidation of sulfides to sulfoxides and sulfones is described in the literature (D. S. Tarbell, C. Weaver, *J. Am. Chem. Soc.*, **1941**, *63*, 2939; I. Sircar et al., *J. Med. Chem.*, **1985**, *28*, 1405). Compounds of Formula Ib are dissolved in an inert solvent such as dichloromethane or benzene and an oxidant such as *meta*-chloroperoxybenzoic acid or monoperphthalic acid is added (Step 1). The reaction can be conducted at a temperature from 0° to 100°C. When the reaction is complete, the reaction mixture is added to a water-immiscible solvent, washed sequentially with aqueous sodium thiosulfate solution, aqueous sodium bicarbonate solution, and water. The organic layer is dried and the solvent is evaporated to yield compounds of Formula Ic.

Following the same procedure, the preparation of sulfones of Formula Id can can be achieved either from sulfoxides of Formula Ic (Step 2), or from compounds of Formula Ib with the use of excess oxidant (Step 3).

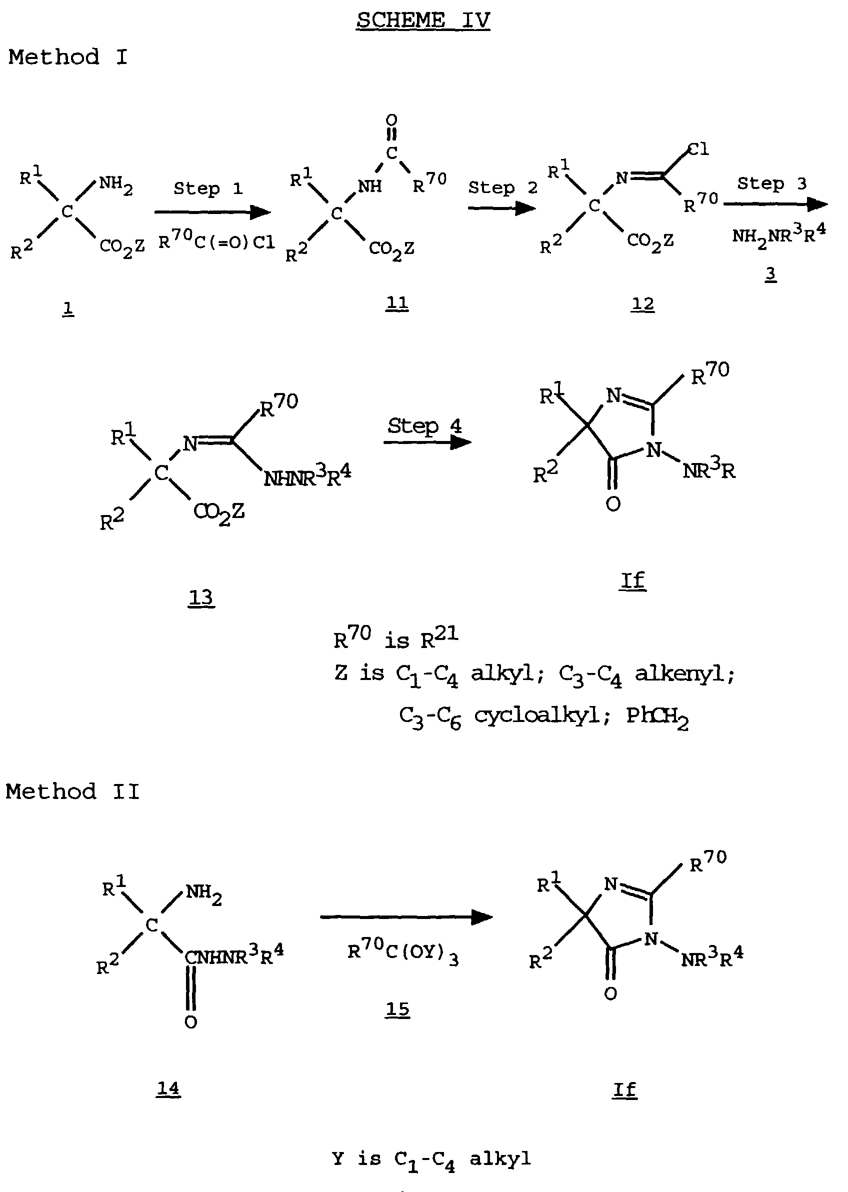

The 4H-imidazol-4-one compounds of Formula If can be prepared by one or more of the methods shown in Scheme IV. The preparation of compounds of Formula 11 from the amino acids of Formula 1 or their salts (Step 1, Method I) can be accomplished using known procedures in the literature [C. A. Buehler, D. E. Pearson, Survey of Organic Syntheses, Vol. 2, p 813, John Wiley and Sons (1977); V. Prelog, P. Wieland, *Helv. Chim. Acta*, **1946**, *29*, 1128]. The preparation of the iminochlorides of Formula 12 (Step 2) from the amides of Formula 11 can be accomplished by literature methods (J. W. Williams et al., *Org. Synth.*, **1946**, *26*, 97; C. C. Price, B. H. Velzar, *J. Org. Chem.*, **1947**, *12*, 386). The displacement of the chloride in the iminochlorides with the hydrazines of Formula 3 (Step 3) can also be achieved by procedures known in the literature (H. Priewe, A. Polzak, *Ber. Deutsch. Chem. Ges.*, **1955**, *88*, 1932; J. Berger et al., *Monatsch. Chem.*, **1981**, *112*, 959). The cyclization of compounds of Formula 13 to heterocycles of Formula If (Step 4) can be accomplished by literature procedures known for the cyclization of *N*-(aminomethylidene)-α-amino acids to 4H-imidazolin-4-ones (Y. Ito et al., *Synth. Commun.*, **1974**, *4*, 289; J. R. Ross et al., *J. Heterocycl. Chem.*, **1987**, *24*, 661)

Alternatively, the compounds of Formula If can be prepared by the procedure illustrated in Method II, Scheme IV. The hydrazides of Formula 14 can be prepared by procedures known in the literature (M. Brenner, W. Hofer, *Helv. Chim. Acta*, **1961**, *44*, 1798; Unit Ika KK, JP 246,362). The condensation of compounds of Formula 14 with ortho-esters of Formula 15 provides compounds of Formula If. For example, an amino-hydrazide of Formula 14 is mixed with an orthoester of Formula 15, in the presence of a catalytic amount of acid, such as acetic acid, at a temperature from 50° to 200°C. When the reaction is complete, the mixture is purified to provide the product of Formula If. The following literature procedures describe the preparation of 4H-imidazol-4-ones from α-amino carboxylic acid amides and ortho-esters (S. Ginsburg, *J. Org. Chem.*, **1962**, *27*, 4062; J. Brunken, G. Bach, *Ber. Deutsch. Chem. Ges.*, **1956**, *89*, 1363). The ortho-esters of Formula 15 can be prepared according to processes described in the literature [C. A. Buehler and D. E. Pearson, Survey of Organic Syntheses, Vol. 2, p 711, John Wiley and Sons (1977)].

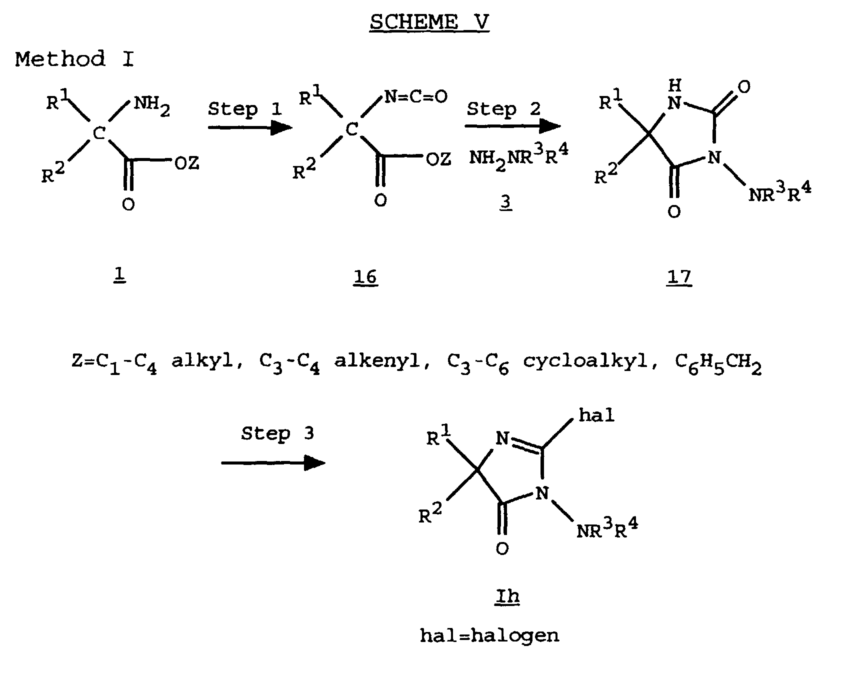

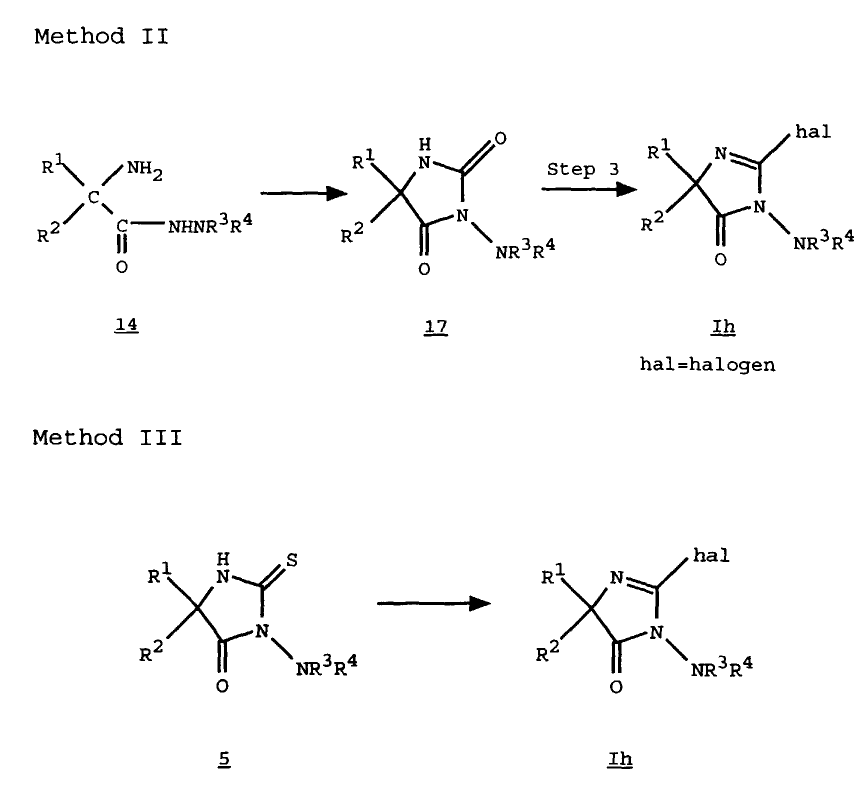

The compounds of Formula Ih can be prepared by one or more of the methods illustrated in Scheme V. The isocyanates of Formula 16 can be prepared (Step 1, Method I) by literature methods, using reagents such as phosgene (L. C. Raitord, H. B. Freyermuth, *J. Org. Chem.*, **1943**, *8*, 230; G. Losse, W. Godicke, *Ber. Deutsch. Chem. Ges.*, **1967**, *100*, 3314), *N,N'*-carbonyldiimidazole (H. A. Staab, W. Benz, *Angew. Chem.*, **1961**, *73*, 66), or oxalyl chloride (V. Von Gizycki, *Angew. Chew.*, **1971**, *83*, 406; M. W. Gittos et al., *J. Chem. Soc., Perkin Trans. 1,* **1976**, 141). The compounds of Formula 17 can be prepared by mixing the isocyanates of Formula 16 and hydrazines of Formula 3 in an inert solvent such as chlorobutane or tetrahydrofuran at a temperature of 0° to 50°C (Step 2). When the reaction is complete, the reaction mixture is poured into an organic solvent and washed sequentially with a dilute aqueous mineral acid solution, a dilute aqueous basic solution, and water. The organic fraction is dried and the solvent is evaporated to give a residue which is redissolved in an inert solvent such as toluene and heated at a temperature of 60° to 200°C. When the cyclization is complete, the solvent is removed to provide compounds of Formula 17. The preparation of 4(H)-imidazol-4-ones of Formula Ih from compounds of Formula 17 (Step 3) can be accomplished using literature procedures known to convert NHC(=O) groups to N=C(halogen) groups (D. Harrison et al., *J. Chem. Soc.*, **1963**, 2930; R. Appel et al., *Ber. Deutsch. Chem. Ges.*, **1974**, *107*, 698; H. V. Dobeneck, T. Messerschmitt, *Liebigs Ann. Chem.,* **1971**, *751*, 32; G. Rio, D. Masure, *Bull. Soc. Chim. Fr.*, **1972**, 4604).

Compounds of Formula 17 can also be prepared from compounds of Formula 14 (Method II, Scheme V) using procedures known in the literature for the preparation of hydantoins from α-amino carboxylic acid amides (S. Goldschmidt, M. Wick, *Liebigs Ann. Chem.*, **1952**, *575*, 217). Conversion of hydantoins of Formula 17 to heterocycles of Formula Ih is discussed in Step 3 above.

Another method to prepare compounds of Formula Ih is illustrated in Method III, Scheme V. This conversion can be accomplished using literature procedures (C. R. Petrie et al., *J. Med. Chem.*, **1985**, *28*, 1010; R. E. Holmes, R. K. Robins, *J. Am. Chem. Soc.*, **1964**, *86*, 1242; R. T. Koda, J. A. Biles, W. Wolf, *J. Pharm. Sci.*, **1968**, *57*, 2056) known for the conversion of NHC(=S) groups to the corresponding N=C (halogen) groups.

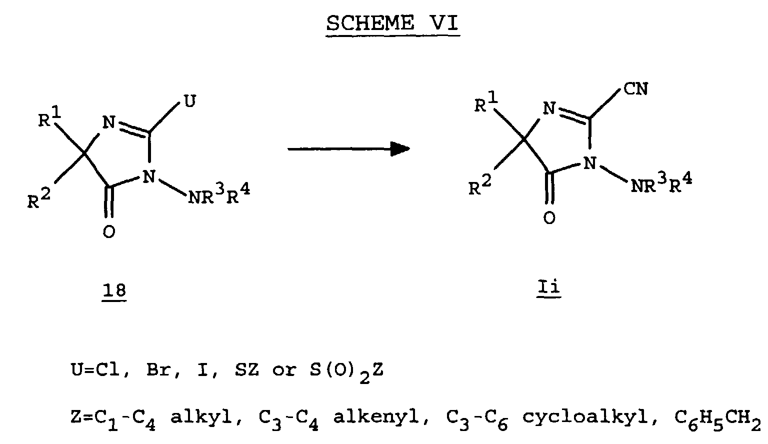

The cyano compounds of Formula Ii can be prepared according to literature procedures for the conversion of -N=C-U functionalities, wherein U=Cl, Br, I, SZ, S(O)₂Z and Z is defined as described above, to -N=C-C≡N moieties, by displacing the group U with cyanide salts. For example, a sulfonyl compound of Formula 18 (U=S(O)₂Z) is treated with a salt of cyanide, such as potassium cyanide, in an inert solvent, such as dimethysulfoxide, at a temperature from 20° to 200°C. When the reaction is complete, the reaction mixture is poured into water and extracted with an organic solvent. The organic extracts are combined, and washed with water. The solvent is removed to provide a product of Formula Ii after purification. (Scheme VI, N. G. Clark, E. Cawkill, *Tetrahedron Lett.*, **1975**, 2717; T. Kato et al., *Chem. Pharm. Bull.*, **1986**, *34*, 3635; P. A. Wade et al., *J. Org. Chem.*, **1983**, *48*, 1796).

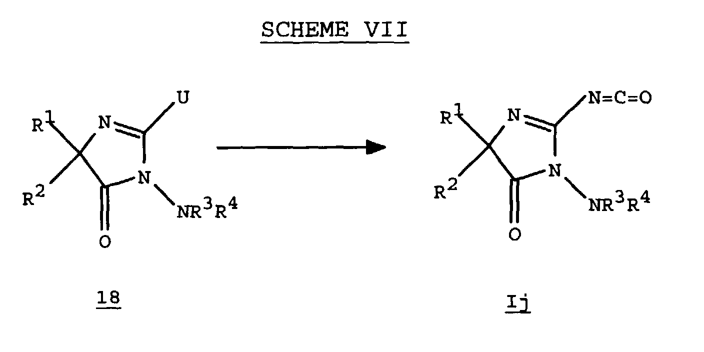

Compounds of Formulae Ij can be prepared as illustrated in Scheme VII. Compounds of Formula Ij can be prepared by displacement of leaving group U with sources of isocyanate, such as the sodium or potassium salt of isocyanate. For example, to a salt of isocyanate, such as potassium isocyanate, in an inert solvent, such as dichloromethane, at a temperature of 20° to 150°C, a compound of Formula 18 is added, with or without the use of a catalytic amount of crown ether, such as 18-crown-6 ether, or a phase transfer reagent, such as tetra-*n*-butylammonium iodide. When the reaction is complete, the solvent is removed and the residue is purified to yield an isocyanate of Formula Ij. (J. Geordeler, R. Richter, *Synthesis*, **1978**, 760).

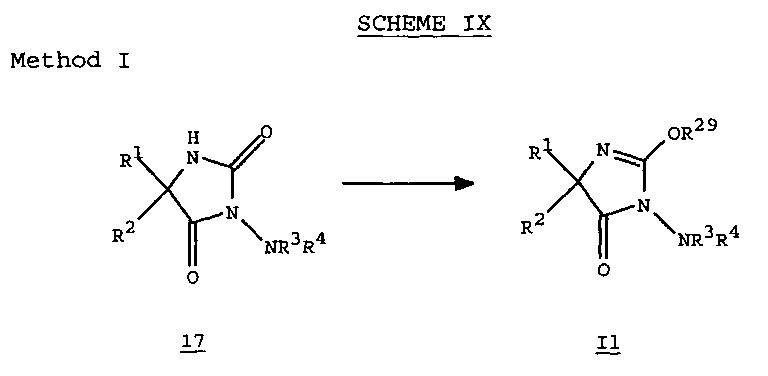

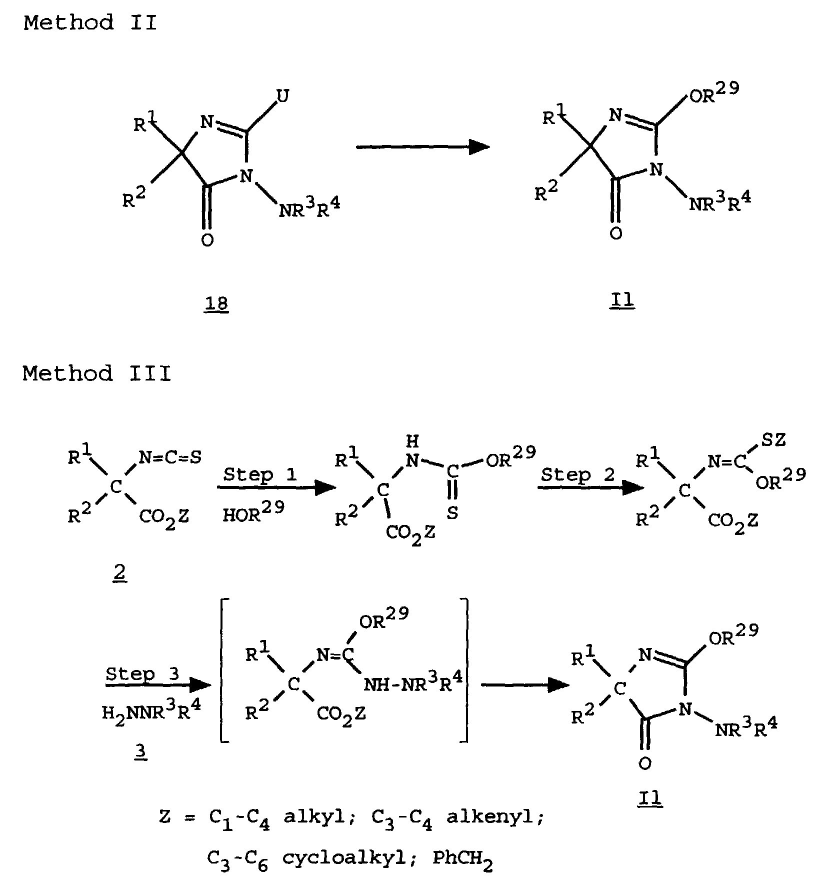

As illustrated in Method I, Scheme IX, 4(H)-imidazol-4-ones of Formula Il can be prepared by reaction of compounds of Formula 17 with reactive electrophiles such as alkyl sulfates (R. Menezes, M. B. Smith, *Synth. Commun.*, 1988, *18*, 1625; G. H. Rasmusson et al., *J. Med. Chem.*, **1984**, *27*, 1690); alkyl halides (D. Libermann et al., *C. R. Acad. Sci.*, **1953**, *237*, 338; S. K. Sihka et al., *Indian J. Chem. B*, **1985**, *24*, 1035; G. Klein et al., *Helv. Chim. Acta*, **1955**, *38*, 1412); alkyl sulfonates (W. Ried, E. Schmidt, *Liebigs Ann. Chem.*, **1965**, *676*, 114, J. A. Warshaw et al., *J. Org. Chem.*, **1989**, *54*, 1736); activated carbonyl compounds, such as carboxylic acid halides (R. R. Koganty, G A. Digenis, *J. Labelled Compounds,* **1974**, *10*, 419; M. Miyake et al., *Synth. Commun.*, **1984**, *14*, 353), isocyanates (W. Logel, K-H. Pook, *Chem. Rev.*, **1986**, *119*, 2553); or diazoalkanes (H. Nishiyama et al., *Tetrahedron Lett.*, **1979**, 4671).

In addition, compounds of Formula Il can be prepared as illustrated in Method II, Scheme IX by displacing the group U in compounds of Formula 18 with nucleophiles. For example, to a stirred solution of a salt of an alcohol, such as sodium ethoxide, in the corresponding alcohol, in this case, ethanol, or in an inert solvent, such as dimethylformamide, a compound of Formula 18 is added. The reaction is carried out at a temperture of 20° to 200°C. When the reaction is complete, the solvent is removed and the residue purified to provide a compound of Formula Il. (E. C. Tayler, F. Koenzle, *J. Org. Chem.*, **1971**, *36*, 233; S. E. Forman et al., *J. Org. Chem.*, **1963**, *28*, 2653; P. A. Wade et al., *J. Org. Chem.*, **1983**, *48*, 1796; M. Mori, *Synthesis*, **1987**, 278).

In addition, compounds of Formula Il can be prepared by the methods illustrated in Method III, Scheme IX. The isothiocyanates of Formula 2 can be condensed with alcohols of Formula HOR²⁹ using literature procedures to form the corresponding thionocarbamates (E. Campaign, P. K. Nargund, *J. Org. Chem.*, **1964**, *29*, 224; G. S. Skinner, H. C. Vogt, *J. Amer. Chem. Soc.*, **1955**, *77*, 5440). The thionocarbamates can be alkylated as illustrated in Step 2 using known methods (R. Gompper, *Ber. Deutsch. Chem. Ges.*, **1956**, *89*, 762; M. Kulka, *Can. J. Chem.*, **1982**, 47, 1985). Compounds of Formula Il can be prepared from treatment of the alkylated thionocarbamate with hydrazines of Formula 3 as illustrated in Step 3. In some instances, the intermediate acyclic compound can be isolated and cyclized in a separate step. Example 3 below describes this synthetic sequence in detail.

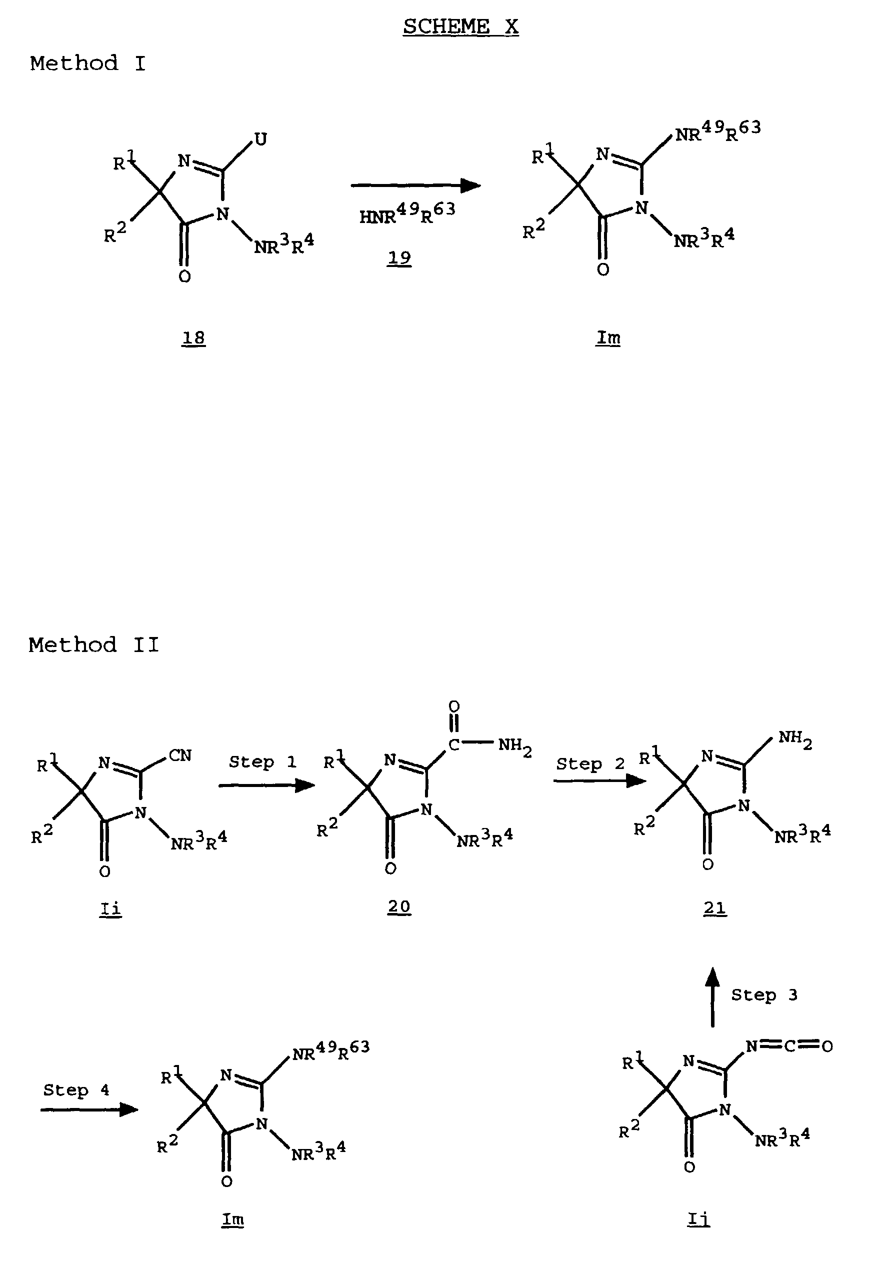

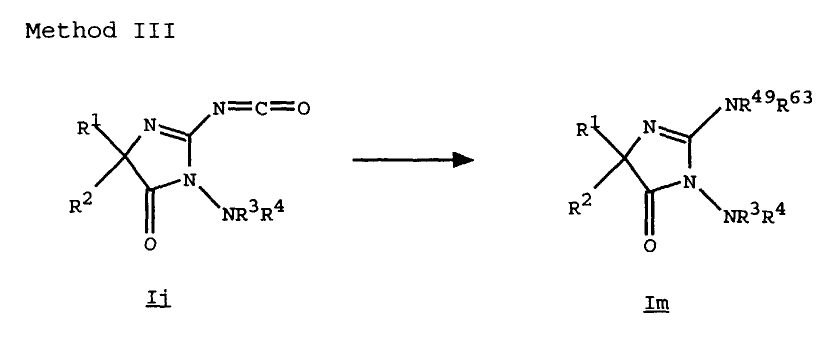

The compounds of Formula Im can be prepared by one or more of the methods illustrated in Scheme X. One method of preparing compounds of Formula Im involves the displacement of group U in compounds of Formula 18 with compounds containing a nitrogen bearing at least one hydrogen (Method I) such as ammonia and its salts (A. V. N. Reddy et al., *Synthesis*, **1986**, 864; A. Yamazaki et al., *J. Org. Chem.*, **1967**, *32*, 3032, 3258; T. Ravindranathan et al., *Org. Prep. Proced. Int.*, **1986**, *18*, 95); primary amines of Formula 19 (M. J. S. Dewar, *J. Chem. Soc.*, 1944, 534; L. Fishbein, J. A. Gallaghan, *J. Am. Chem. Soc.*, **1954**, *76*, 1877); and secondary amines of Formula 19 (S. R. Aspinall, E. J. Bianco, *J. Am. Chem. Soc.*, **1951**, *73*, 602; ibid., **1957**, *79*, 2199). For example, to a stirred solution of a compound of Formula 18 in an inert solvent such as chloroform, at a temperature of 0° to 150°C, an amino compound of Formula 19 is added. When the reaction is complete, solvent is removed and the residue thus obtained is purified to give a compound of Formula Im.

Alternatively, compounds of Formula Im can be prepared as illustrated in Method II, Scheme X. The compounds of Formula 21 can be prepared by one or both of the procedures shown. Compounds of Formula Ii (for the preparation of Ii, see Scheme VI) can be hydrolyzed (Step 1) by literature methods to amides of Formula 20 [C. A. Buehler, D. E. Pearson, Survey of Organic Synthesis, Vol. **2**, 813, John Wiley and Sons (1977); H. O. Larson et al., *J. Org. Chem.*, **1969**, *34*, 525] which in turn can be converted (Step 2) to compounds of Formula 21 by methods described in the literature (R. C. Jones, *J. Am. Chem. Soc.*, **1951**, *73*, 5610; J. Buchi et al., *Helv. Chim. Acta*, **1949**, *32*, 1806; F. J. McCarty et al., *J. Med. Chem.*, **1970**, *13*, 814). Another way to prepare compounds of Formula 21 is to hydrolyze (Step 3) compounds of Formula Ij using procedures known in the literature (J. Weinstock, *J. Org. Chem.*, **1961**, *26*, 3511; A. Hassner, C. Heathcock, *Tetrahedron*, **1964**, *20*, 1037). Compounds of Formula Im can be prepared from compounds of Formula 21 by derivatization of the amino group at the 2-position of the 4H-imidazol-4-ones of Formula 21 (Step 4) with reactions such as alkylations (A. M. Monro, *Chem. Ind.*, **1964**, 1806; P. Nesbitt, P. Sykes, *J. Chem. Soc.*, **1954**, 3057; P. Molina et al., *Chem. Ber.*, **1988**, *121*, 1495).

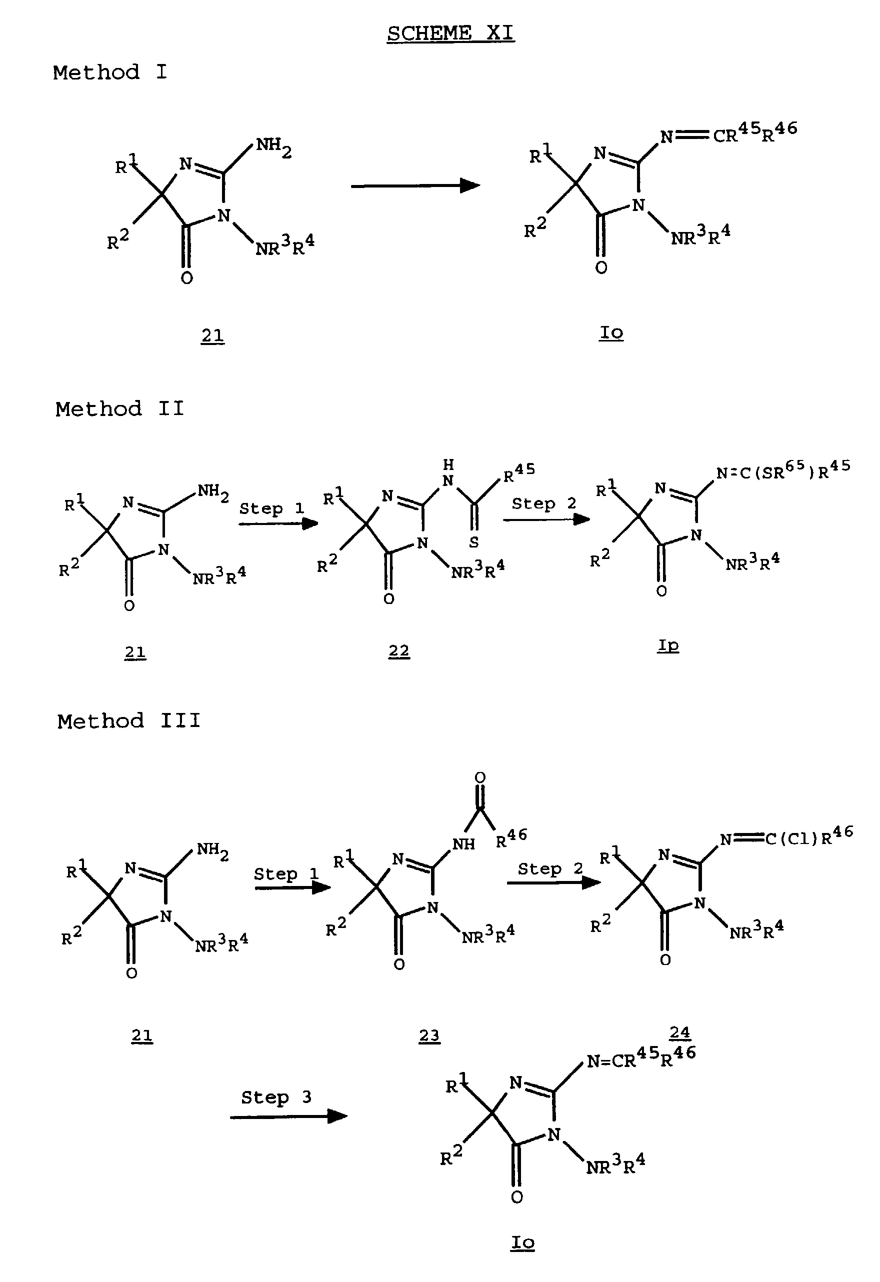

The compounds of Formula Io can be prepared by one or more of the methods illustrated in Scheme XI. As shown in Method I, the compounds of Formula Io can be obtained from amino compounds of Formula 21 by condensation with carbonyl compounds of formula R⁴⁵R⁴⁶C=O (H. Van Der Poel, G. Van Koten, *Synth. Commun.*, **1978**, *8*, 305; M. Chaykowsky et al., *J. Heterocycl. Chem.*, **1977**, *14*, 661), or ortho-esters of carboxylic acids of formula R⁴⁵C(OR⁶⁵)₃ to form compounds of Formula Io wherein R⁴⁶=OR⁶⁵. For example, an amino compound of Formula 21 is stirred with an ortho-ester, with or without the use of solvent, at a temperature of 30° to 200°C. When the reaction is complete, the reaction mixture is purified to provide a compound of Formula Io. (R. A. Crochet, Jr., C. D. Blanton, Jr., *Synthesis*, **1974**, 55; F. M. F. Chen, N. L. Benoiton, *Can. J. Chem.*, **1977**, *55*, 1433). The carbonyl compounds and the ortho-esters can be prepared by literature procedures. For carbonyl compounds: C. A. Buehler, D. E. Pearson, Survey of Organic Synthesis, Vol. **2**, pp 480, 532; John Wiley and Sons (1977); for ortho-esters: ibid., p 711.

The compounds of Formula Ip can be prepared using the procedure illustrated in Method II, Scheme XI. The compounds of Formula 22 can be prepared from the amino compounds of Formula 21 by literature methods (Step 1, G. C. Barrett, C. M. O. A. Martins, *J. Chem. Soc. Chem. Commun.*, **1972**, 698; W. Walter, M. Radke, *Liebigs Ann. Chem.*, **1970**, *739*, 201; P. Schlack, G. Keil, *Liebigs Ann. Chem.*, **1963**, *661*, 164; W. Reid, W. Vond Der Emden, *Liebigs Ann. Chem.*, **1961**, *642*, 128). Compounds of Formula Ip can be obtained from compounds of Formula 22 by known methods. For example, to a stirred solution of a compound of Formula 22 in an inert solvent, such as dichloromethane, at a temperature of 20° to 100°C, an alkyl halide, such as iodomethane is added. When the reaction is complete, the solvent is removed to provide, after purification, a compound of Formula Ip. (Step 2, H. Nishiyama et al., *Tetrahedron Lett.*, **1979**, 4405; M. Bercot-Vatteroni, *Liebigs Ann. Chem.*, **1962**, *7*, 312; A. A. Goldberg, W. Kelly, *J. Chem. Soc.*, **1948**, 1919).

Another method for the preparation of compounds of Formula 1o is illustrated in Method III, Scheme XI. Following literature methods, the compounds of Formula 23 can be prepared from compounds of Formula 21 (Step 1). For R⁴⁶C(=O)N as amides: S. Sandler, W. Karo, Organic Functional Group Preparations (Second Edition), Vol. **1**, p 316, Academic Press (1983); for R⁴⁶C(=O)N as carbamates: ibid., (First Edition), Vol 2, p 223, Academic Press (1971); for R⁴⁶C(=O)N as thiolcarbamate: A. Hajos, *Ber. Deutsch. Chem. Ges.*, **1961**, *94*, 2350; G. Harris, I. C. MacWilliam, *J. Chem. Soc.*, **1961**, 2053. Compounds of Formula 24 can be prepared from compounds of Formula 23 (Step 2) by literature methods (R. Appel et al., *Ber. Deutsch. Chem. Ges.*, **1974**, *107*, 698; K. Fijimoto et al., *Chem. Ind.*, **1971**, 175; W. Reid, R. Christ, *Liebigs Ann. Chem.*, **1980**, 693). The compounds of Formula Io can be obtained from compounds of Formula 24 according to literature methods with the use of reagents such as carbon nucleophiles (H. Quast et al., *Liebigs Ann. Chem.*, **1979**, *83*; N. G. Clark, E. Cawkill, *Tetrahedron Lett.*, **1975**, 2717; W. Reid, P. Weidemann, *Ber. Deutsch. Chem. Ges.*, **1971**, *104*, 3329).

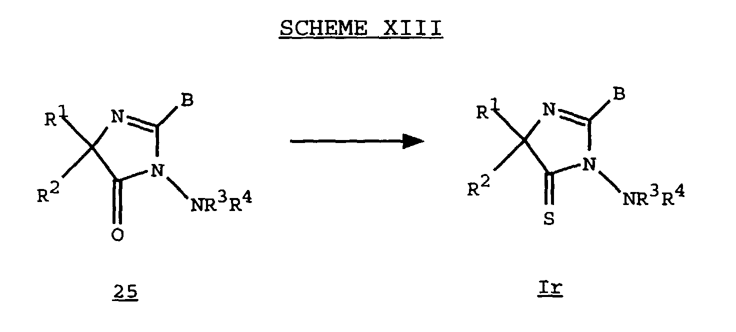

Compounds of Formula Ir can be prepared from compounds of Formula 25, as illustrated in Scheme XIII, following literature methods used to prepare thioamides and thioureas from the corresponding amides and ureas, respectively, using reagents such as sulfur, P₄S₁₀, or Lawesson's reagent. For example, to a solution of a compound of Formula 25 in an inert organic solvent such as benzene or acetonitrile, a solution of a sulfurizing reagent, such as P₄S₁₀ in the same solvent is added, followed by an inorganic salt, such as sodium bicarbonate. The reaction temperature is maintained at 0° to 100°C. When the reaction is complete, an organic solvent, such as diethyl ether is added and the mixture is washed with a basic aqueous solution, such as sodium bicarbonate and then water. The solvent is removed to give, after purification, a compound of Formula Ir. (H. Alper et al., *Angew. Chem., Int. Ed. Eng.*, **1978**, *17*, 689; J. Perregaard et al., *Bull. Soc. Chim. Belg.*, **1977**, *86*, 321; J. Voss, *Liebigs Ann. Chem.*, **1971**, *746*, 92; T. Nishio et al., *Synthesis*, **1989**, 396; J. M. Kane, *Synthesis*, **1987**, 912)

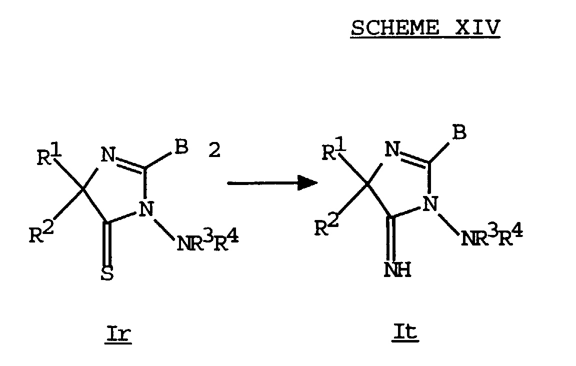

Compounds of Formula It can be prepared as illustrated in Scheme XIV. Compounds of Formula It can be obtained from compounds of Formula Ir by treatment with sources of ammonia. For example, to a stirred solution of a compound of Formula Ir in an inert solvent such as dichloromethane, a source of ammonia, such as ammonia gas or ammonium chloride, is added at a temperature of -20° to 50°C. When the reaction is complete, the solvent is removed to provide, after purification, a compound of Formula It. (Step 1, G. S. Skinner, H. C. Vogt, *J. Am. Chem. Soc.*, **1955**, *77*, 5440; F. H. S. Curd et al., *J. Chem. Soc.*, **1948**, 586; J. S. Morley, J. C. E. Simpson, *J. Chem. Soc.*, **1952**, 2617).

### EXAMPLE 1

### Preparation of 3,5-dihydro-5-methyl-2-methylthio-5-phenyl-3-(phenylamino)-4H-imidazol-4-one

To a solution of 5-methyl-5-phenyl-3-phenylamino-2-thioxo-4-imidazolidinone (0.1 g, 0.34 mmol) in dichloromethane (10 mL), iodomethane (0.25 g, 1.75 mmol) and triethylamine (0.18 mL, 1.8 mmol) were added. The mixture was stirred for 48 h, poured into water (10 mL), and extracted with ethyl acetate (three times with 25 mL). The organic extracts were combined, washed with water, dried (MgSO₄), and filtered, and the solvent was removed *in vacuo* to give the title compound as a slightly yellow solid, (95 mg, 90%), m.p. 132-134°C; ¹H NMR (CDCl₃): δ 1.77 (s, 3H), 2.61 (s, 3H), 6.15 (s, 1H), 6.70-7.62 (m, 10H)

### EXAMPLE 2

### Preparation of 3,5-dihydro-2-methoxy-5-methyl-5-phenyl- 3-(phenylamino)-4H-imidazol-4-one

To a solution of ethyl α-[(methoxythioxomethyl)amino]-α-methyl-benzeneacetate (0.2 g, 0.75 mmol) in dichloromethane (1 mL) was added iodomethane (0.5 g, 3.5 mmol) followed by the dropwise addition of 1,8-diazabicyclo[5.4.0]undec-7-ene (0.25 g, 1.6 mmol). The reaction mixture was stirred for 10 minutes, poured into water (10 mL), and extracted with ethyl acetate (3 times with 10 mL). The organic extracts were combined, washed with water, dried (MgSO₄), and filtered. The solvent was removed in vacuo to give 0.2 g of ethyl α-[[methoxy-(methylthio)methylene]amino]-α-methylbenzeneacetate (95% yield) as an oil. ¹H NMR (CDCl₃): δ 1.25 (t, J=7 Hz, 3H), 1.80 (s, 3H), 2.50 (s, 3H), 4.98 (s, 3H), 4.15 (q, J=7 Hz, 2H), 7.35-7.90 (m, 5H).

To a solution of ethyl α-[[methoxy(methylthio)methylene]amino]-α-methylbenzeneacetate (0.2 g, 0.71 mmol) in glacial acetic acid (1 mL) was added anhydrous phenylhydrazine (0.1 g, 0.93 mmol). The reaction mixture was stirred for 15 minutes, poured into aqueous sodium carbonate, and extracted with ethyl acetate. The ethyl acetate extracts were combined, washed with water, dried (MgSO₄), and filtered. The solvent was removed in vacuo to afford an oil which was purified by silica gel chromatography (ethyl acetate:hexanes = 1:2) to give two fractions. Fraction 1 was ethyl α-[[methoxy(2-phenyl-hydrazino)methylene]amino-α-methylbenzeneacetate (R_{f}=0.33, 40 mg, 16% yield). ¹H NMR (CDCl₃): δ 1.20 (t, J=6.7 Hz, 3H), 2.00 (s, 3H), 3.75 (s, 3H), 4.20 (q, J=6.7 Hz, 2H), 5.50 (bs, 1H), 6.75 (bs, 1H), 6.90-7.70 (m, 10H). Fraction 2 was the title compound (R_{f}=0.07, 10 mg, 4.5% yield). m.p. 110-111°C; ¹H NMR (CDCl₃): δ 1.78 (s, 3H), 4.13 (s, 3H), 6.01 (s, 1H), 6.69-7.67 (m, 10H).

Ethyl α-[[methoxy(2-phenylhydrazino)methylene]amino-α-methylbenzeneacetate was converted into the title compound by heating it for 30 seconds in a round-bottomed flask with a hot-air gun. The compound was purified by silica gel chromatography (ethyl acetate:hexane = 1:2). An additional 23 mg (3.3% yield) was obtained.

### EXAMPLE 3

### Preparation of 3,5-dihydro-5-methyl-2-methylthio-5- heptyl-3-(phenylamino)-4H-imidazol-4-one

To ethyl 2-[[bis(methylthio)methylene]amino]-2-methylnonanoate (4 g, 12.54 mmol) in acetic acid (100 mL) was added phenylhydrazine (1.6 g, 14.8 mmol) and the mixture was stirred for 5 h. The reaction mixture was then slowly poured into enough aqueous sodium carbonate to neutralize the acetic acid. The aqueous mixture was extracted with ethyl acetate. The ethyl acetate extracts were combined, washed with water, dried (MgSO₄), and filtered. The solvent was removed in vacuo to give an oil which was purified by silica gel chromatography (ethyl acetate:hexane = 1:2). The title compound (1.35 g, 32%) was obtained. m.p. 108-110°C, ¹H NMR (CDCl₃): δ 0.86 (t, J=2.4 Hz, 3H), 1.05-1.38 (m, 10H), 1.39 (s, 3H), 1.62 (m, 2H), 2.51 (s, 3H), 6.16 (s, 1H), 6.78-7.25 (m, 5H).

### EXAMPLE 4

### Preparation of 3,5-dihydro-2,5-dimethyl-5-phenyl-3- (phenylamino)-4H-imidazol-4-one

To ethyl α-(acetylamino)-α-methylbenzeneacetate (0.2 g, 0.85 mmol) in carbon tetrachloride (10 mL) was added phosphorus pentachloride (0.26 g, 1.27 mmol). The mixture was stirred and heated at reflux for 5 minutes. The solvent was removed in vacuo. The residue was redissolved in dichloromethane (2 mL) and added dropwise to a solution of phenylhydrazine (0.34 g, 3.15 mmol) in dichloromethane (10 mL). The reaction solution was stirred at room temperature for 24 h, poured into water, and extracted with ethyl acetate. The ethyl acetate extracts were combined, washed with water, dried (MgSO₄), and filtered. The solvent was then removed in vacuo to afford a solid which was recrystallized from 1-chlorobutane to give the title compound (0.15 g, 63.2%). m.p. 110-112°C, ¹H NMR (CDCl₃): δ 1.63 (s, 3H), 2.16 (s, 3H), 6.58-7.50 (m, 10H), 8.69 (s, 1H).

### EXAMPLE 5

### Preparation of 3,5-dihydro-2-methoxy-5-methyl-3- (methyl-phenylamino)-5-phenyl-4H-imidazol-4-one

5-Methyl-5-phenyl-3-(phenylamino)-2,4-imidizolidine-dione(2.5 g, 8.88 mmol), iodomethane (14 mL), and silver oxide (2.6 g, 12.59 mmol) were combined and stirred at room temperature for 2 h. The reaction mixture was filtered, and the solvent was removed in vacuo. The residue obtained was stirred with ethyl acetate. The solid which did not dissolve was filtered off and further washed with ethyl acetate. The ethyl acetate filtrate and washes were combined and evaporated in vacuo to give a residue which was purified by silica gel chromatography (ethyl acetate:hexane = 1:2) to afford the title compound (0.2 g, 7.6% yield). m.p. 183-185°C; ¹H NMR (CDCl₃): δ 1.72 (s, 3H), 3.20 (s, 3H), 4.02 (s, 3H), 6.50-7.80 (m, 10H).

Additional compounds were prepared using the procedures exemplified above in Examples 1-5, and are listed in Index Table A hereinafter.

The compounds referred to in the Tables which follow are illustrated below. The groups R¹-R⁶⁹ and A, B and V are as defined in Formula I in the Summary of the Invention. Additional variables are used in the compounds and Tables and are:
q and r designate the length of some alkoxyalkyl R² groups. The sum of q and r is 2-20.

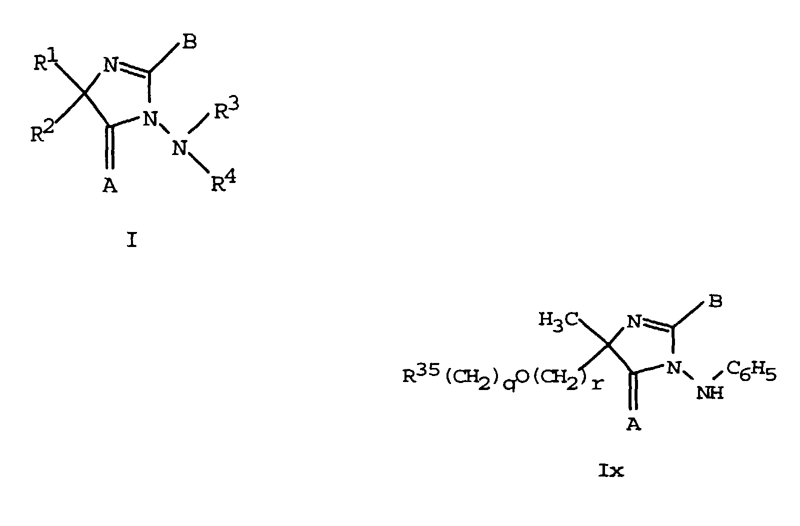

The following abbreviations are used in the Tables which follow. All alkyl groups are the normal isomers unless indicated otherwise.

| | | |
|---|---|---|
| Me = methyl | MeO = methoxy | *t* = tertiary |
| Et = ethyl | EtO = ethoxy | *s* = secondary |
| Ph = phenyl | EtS = ethylthio | *i* = iso |
| Bzl = benzyl | MeS = methylthio | CN = cyano |
| PhCH₂ = benzyl | PhS = phenylthio | *c* = cyclo |

**TABLE 1**

| | | | |
|---|---|---|---|
| Compounds of Formula I wherein A=O, B=MeS, and: | | | |

| R¹ | R² | R³ | R⁴ |
|---|---|---|---|
| Me | 2,4-diF-Ph | Ph | H |
| Et | 2,4-diF-Ph | Ph | H |
| *n*-butyl | 2,4-diF-Ph | Ph | H |
| CF₃ | 2,4-diF-Ph | Ph | H |
| vinyl | 2,4-diF-Ph | Ph | H |
| Me | 2,4-diF-Ph | 4-F-Ph | H |
| Me | 2,4-diF-Ph | 3-F-Ph | H |
| Me | 2,4-diF-Ph | 3-Cl-Ph | H |
| Me | 2,4-diF-Ph | 3,4-diF-Ph | H |
| Me | 2,4-diF-Ph | 2-Me-Ph | H |
| Me | 2,4-diF-Ph | 4-Me-Ph | H |
| Me | 2,4-diF-Ph | 4-NO₂-Ph | H |
| Me | 2,4-diF-Ph | 2-cyano-Ph | H |
| Me | 2,4-diF-Ph | 3-CF₃-Ph | H |
| Me | 2,4-diF-Ph | 3-MeO-Ph | H |
| Me | 2,4-diF-Ph | 4-CF₃O-Ph | H |
| Me | 2,4-diF-Ph | 2-Me-3-Cl-Ph | H |
| Me | 2,4-diF-Ph | 2-Me-3-F-Ph | H |
| Me | 2,4-diF-Ph | 2-F-3-Cl-Ph | H |
| Me | 2,4-diF-Ph | 2,4-diMe-Ph | H |
| Me | 2,4-diF-Ph | Ph | Me |

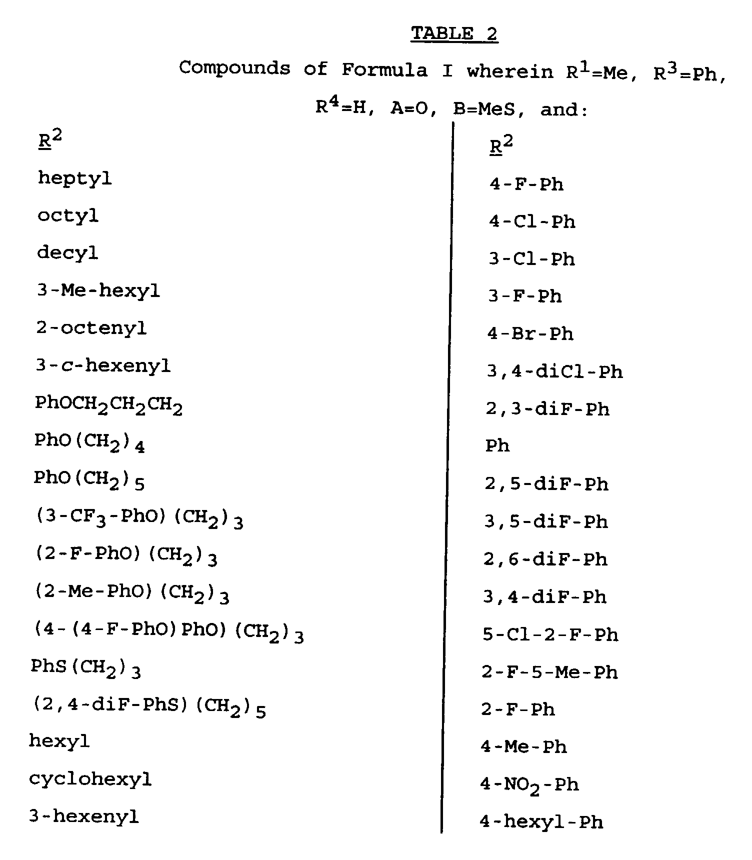

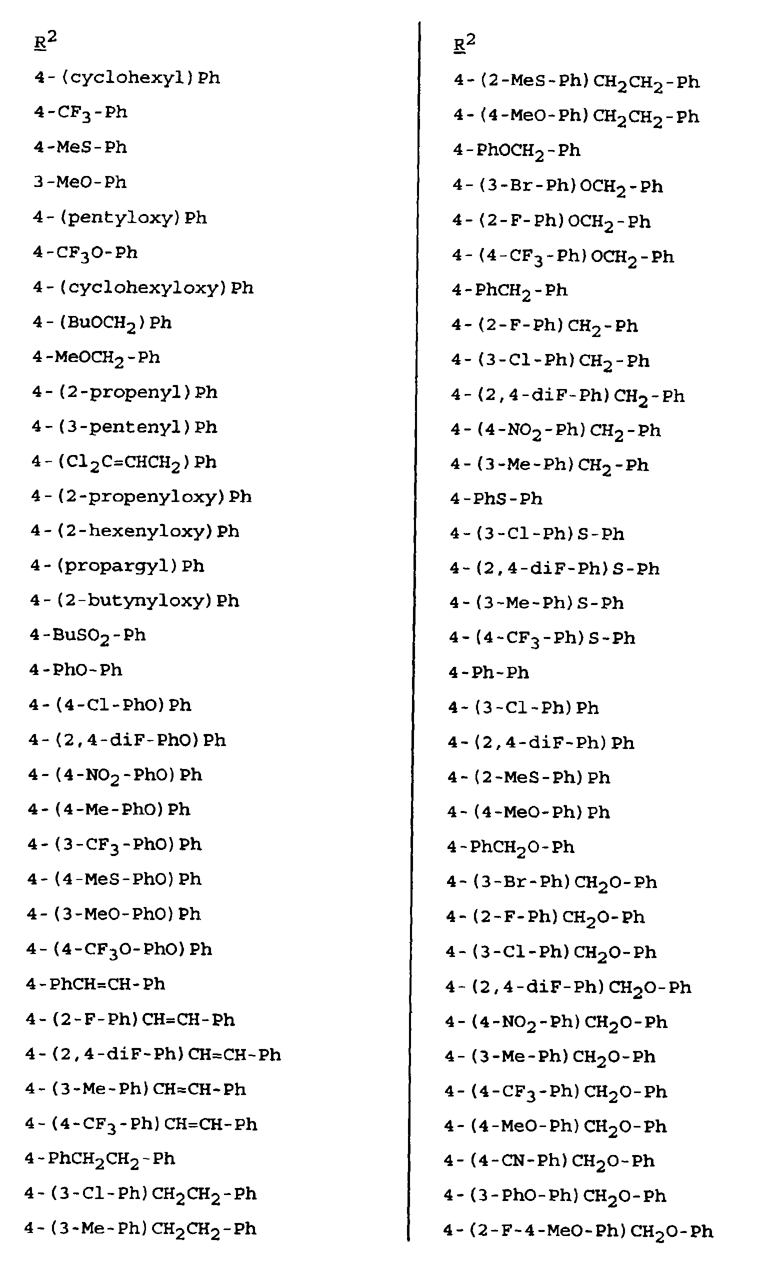

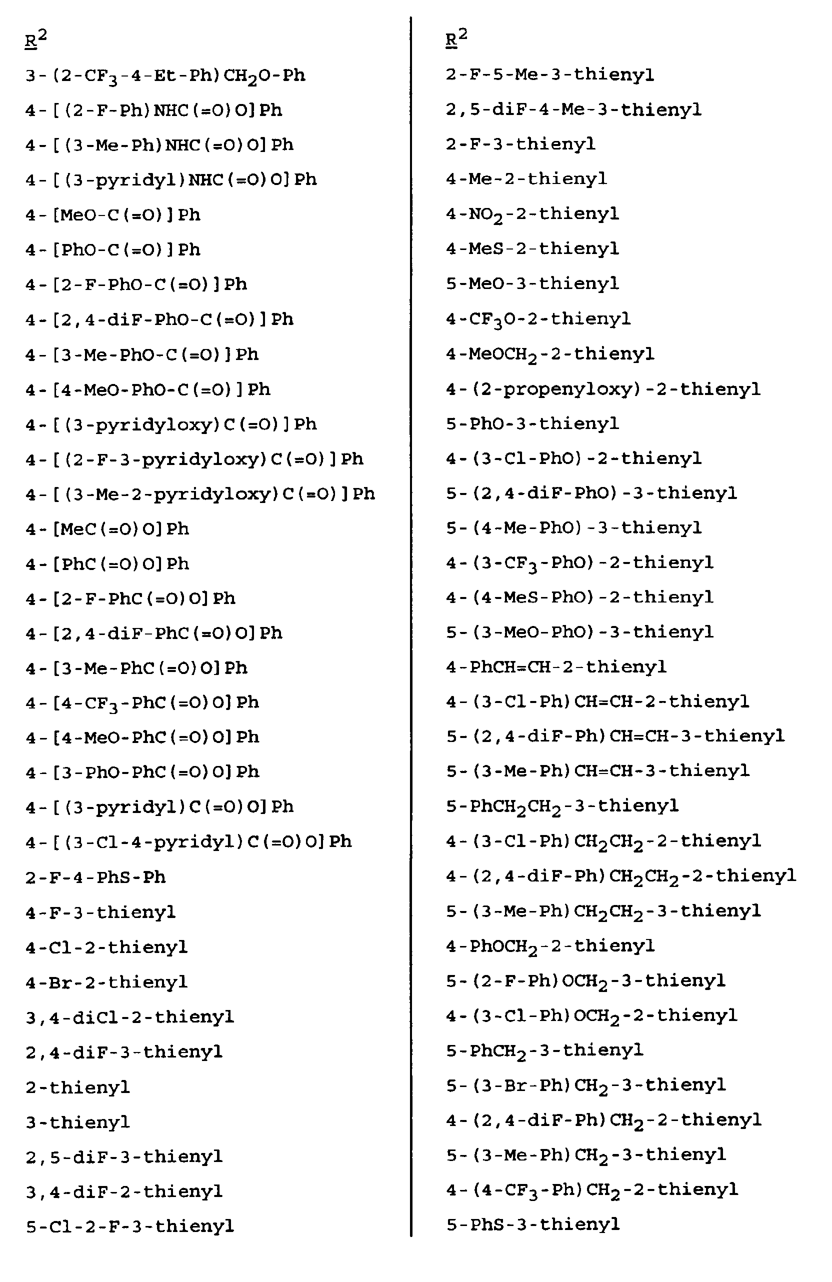

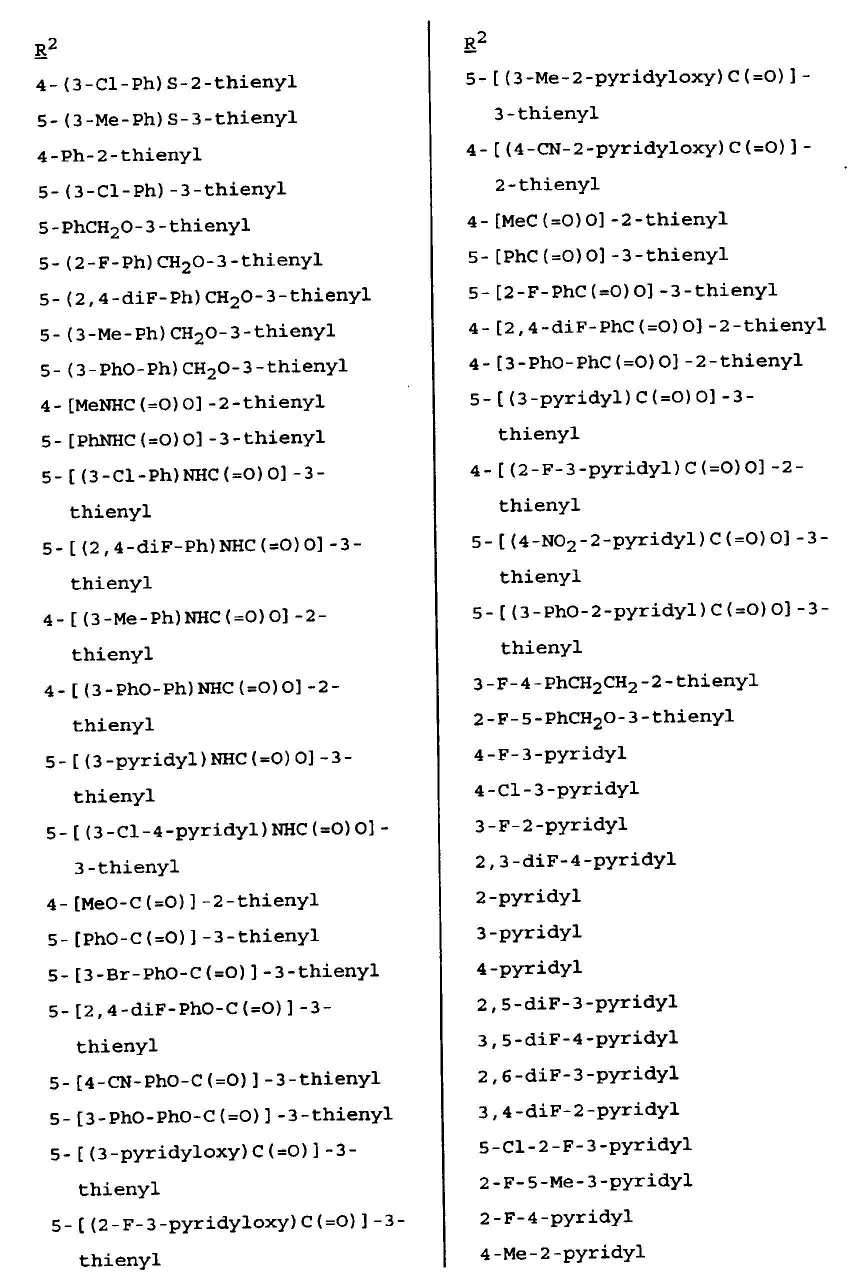

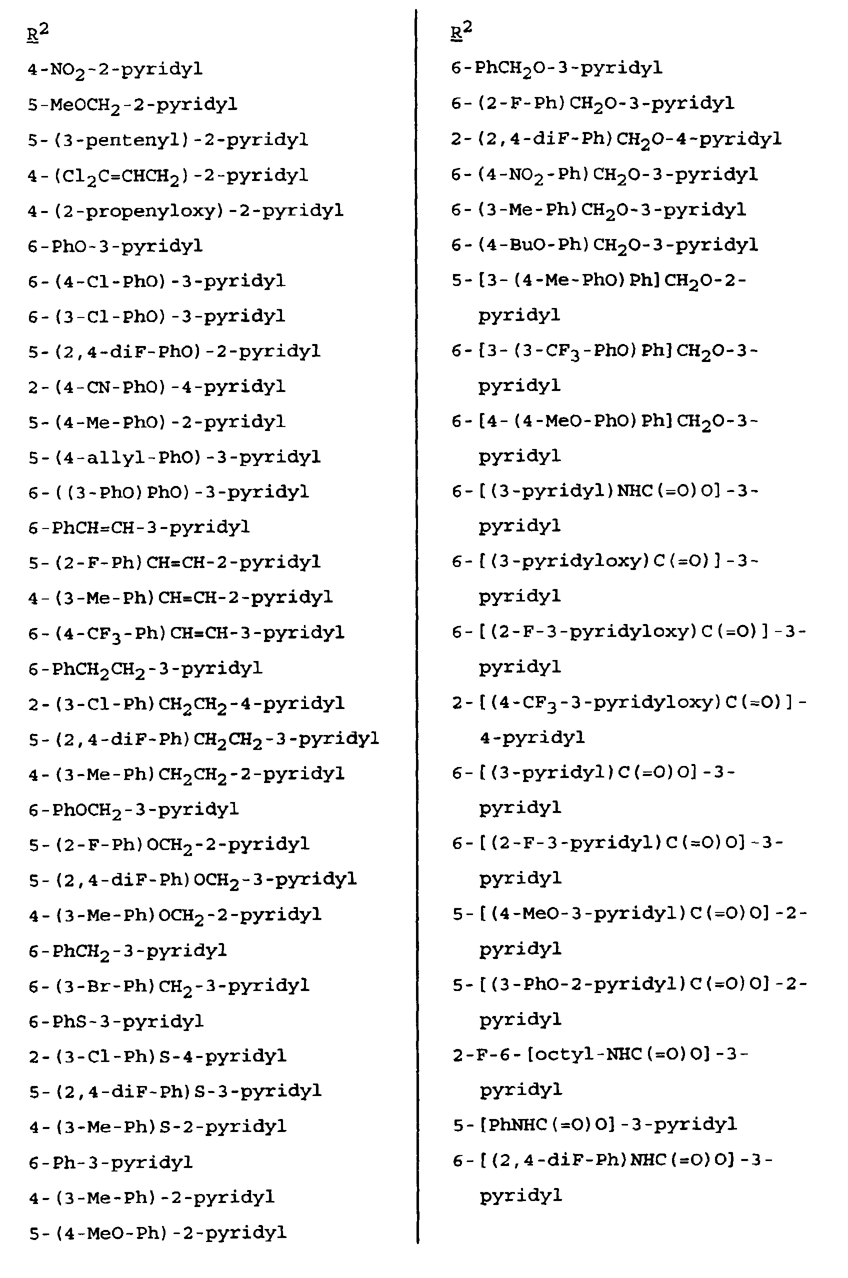

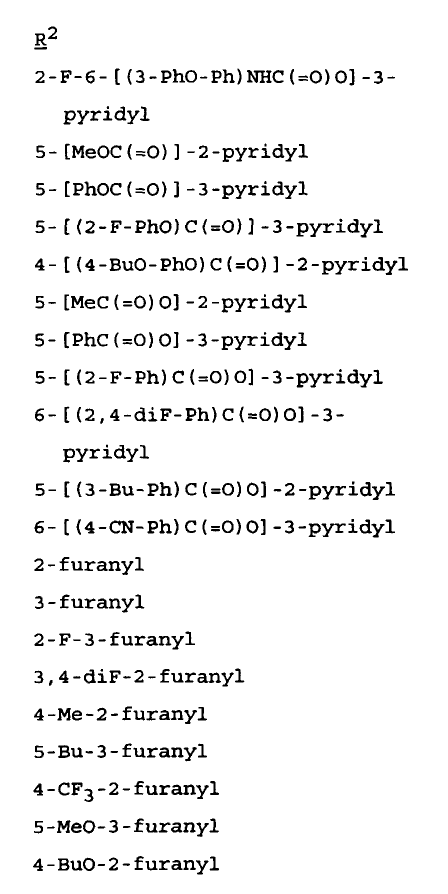

**TABLE 5**

| | | |
|---|---|---|
| Compounds of Formula Ix wherein A=O, B=MeS, and: | | |

| r | q | R³⁵ |
|---|---|---|
| 2 | 6 | H |
| 2 | 8 | H |

**TABLE 6**

| | |
|---|---|
| Compounds of Formula I wherein R¹=Me, R²=Ph, R³=Ph, R⁴=H, A=N-R¹⁵, B=MeS, and: | |

| R¹⁵ | R¹⁵ |
|---|---|
| H | H (PhSO₃H salt) |
| H (HBr salt) | H (4-TsOH salt) |
| H (HCl salt) | H [(4-dodecyl-Ph)So₃H salt] |
| H (HI salt) | H (H₃PO₄ salt) |
| H (CF₃CO₂H salt) | H (camphor sulfonic acid salt) |
| H (MeSO₃H salt) | |

**TABLE 12**

| |
|---|
| Compounds of Formula I wherein R¹=Me, R²=Ph, R³=Ph, R⁴=H, A=N-J, B=MeS, and: |

| J |
|---|
| H |
| H (HBr salt) |

**TABLE 13**

| | |
|---|---|
| Compounds of Formula I wherein R²=Ph, R³=Ph, R⁴=H, A=N-H (HBr salt), B=MeS, and: | |

| R¹ | R¹ |
|---|---|
| Me | CF₃ |
| Et | vinyl |

**TABLE 14**

| | |
|---|---|
| Compounds of Formula I wherein R¹=Me, R²=Ph, R⁴=H, A=N-H, B=MeS, and: | |

| R³ | R³ |
|---|---|
| 4-Cl-Ph | 4-MeO-Ph |
| 4-Br-Ph | 4-MeS-Ph |
| 4-F-Ph | 4-CF₃O-Ph |
| 4-NO₂-Ph | |
| 4-Me-Ph | |
| 4-CF₃-Ph | |

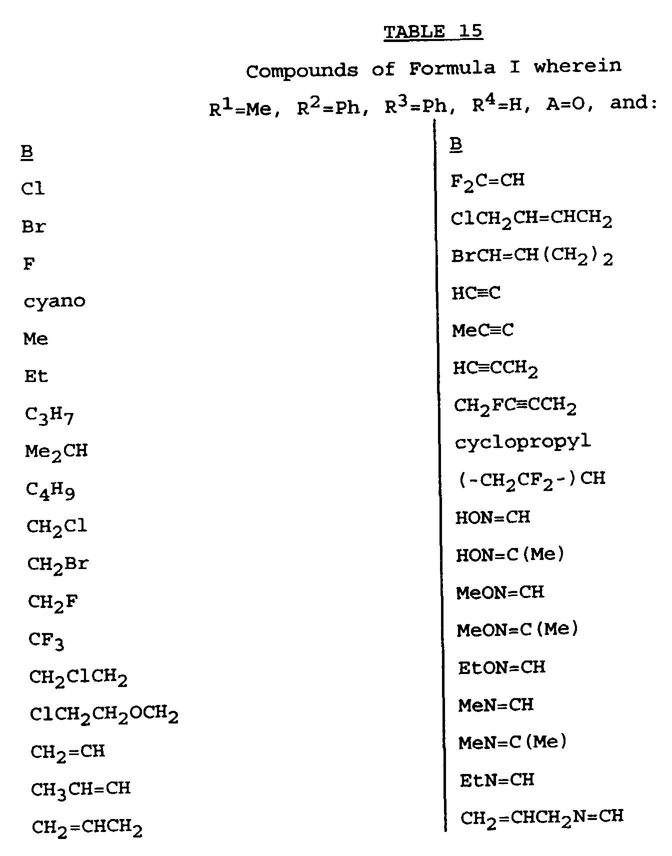

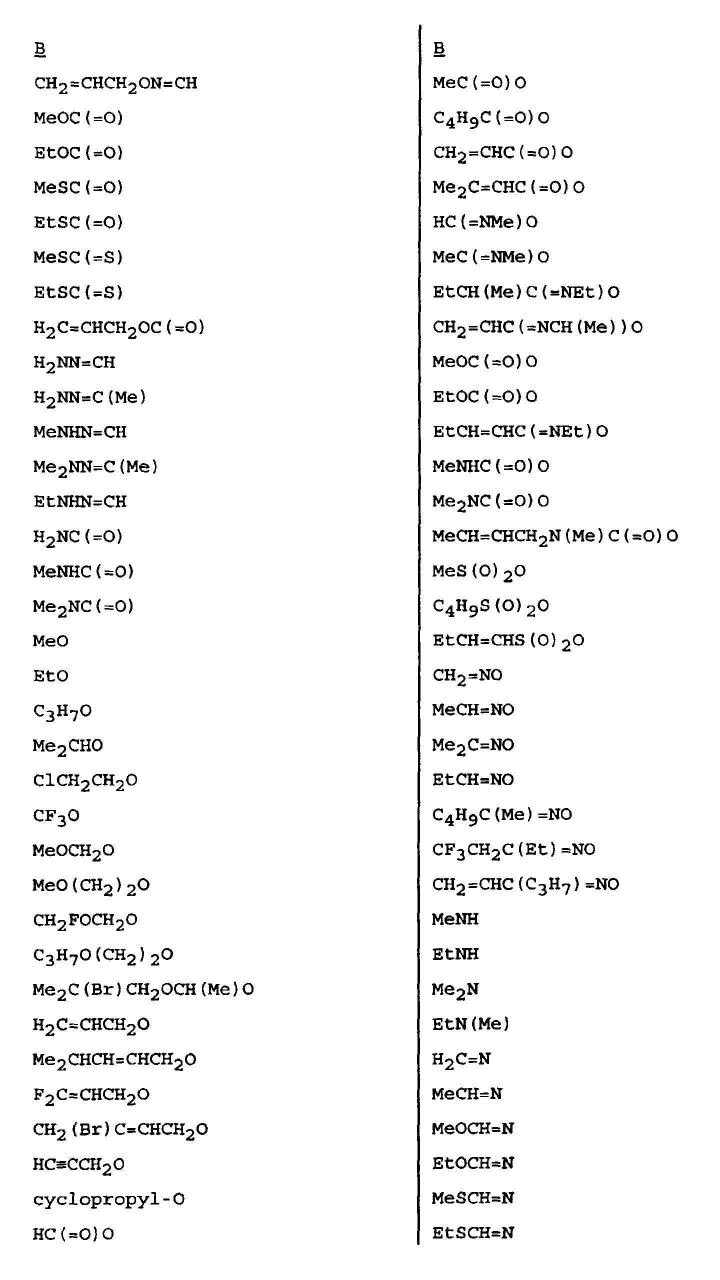

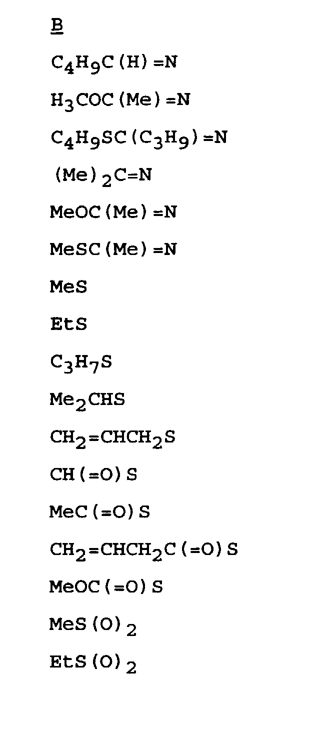

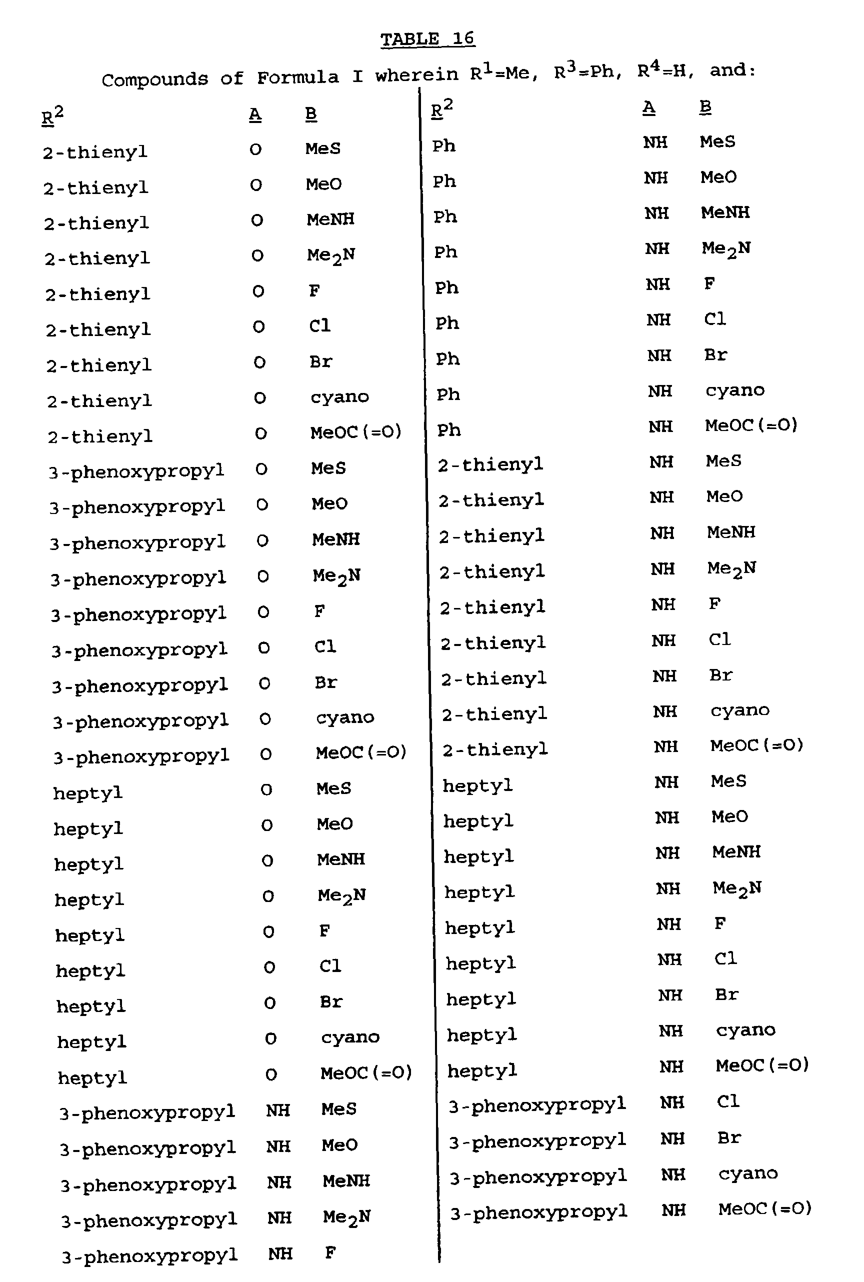

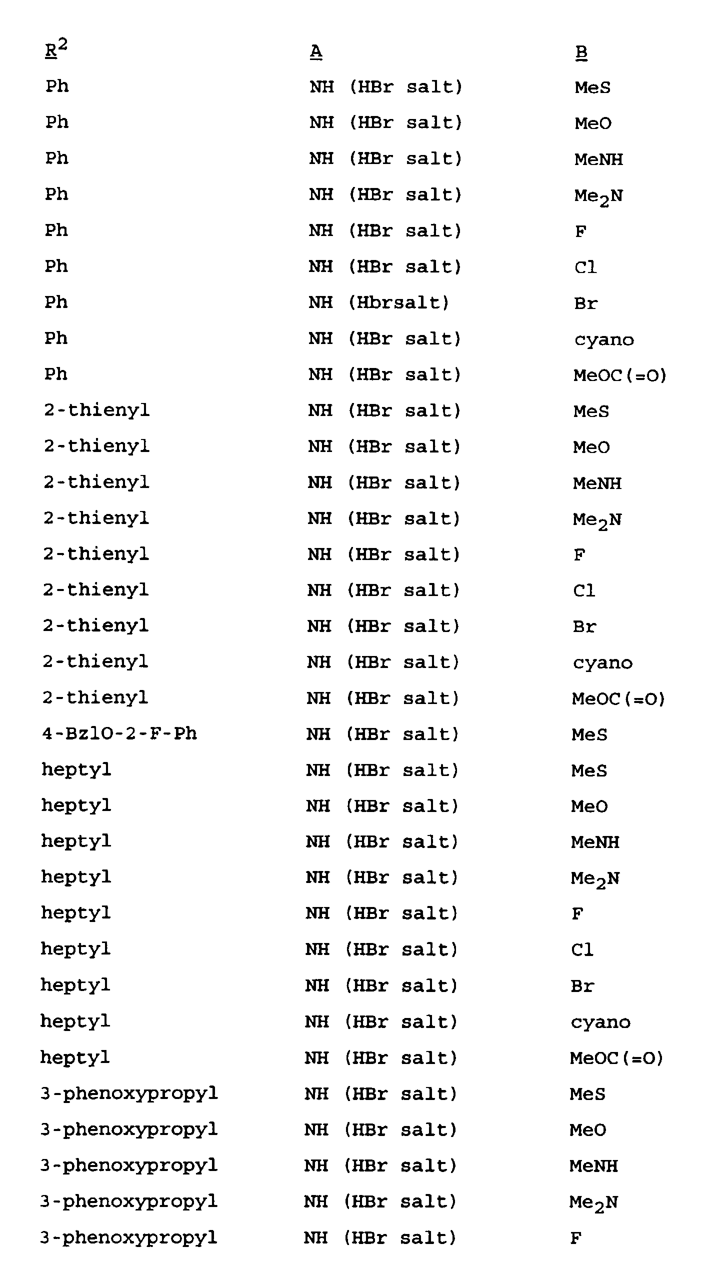

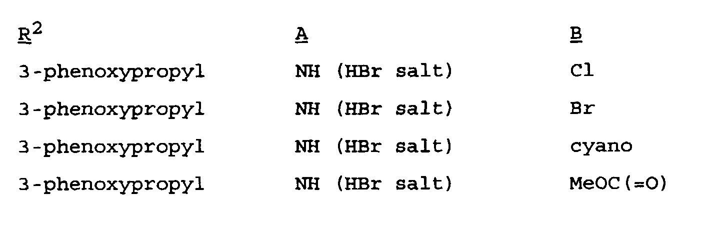

### Formulation/Utility

Compounds of this invention will generally be used in formulation with an agriculturally suitable composition. The fungicidal compositions of the present invention comprise an effective amount of at least one compound of Formula I as defined above and at least one of (a) a surfactant, (b) an organic solvent, and (c) at least one solid or liquid diluent. Useful formulations can be prepared in conventional ways. They include dusts, granules, pellets, solutions, suspensions, emulsions, wettable powders, emulsifiable concentrates, dry flowables and the like. Sprayable formulations can be extended in suitable media and used at spray volumes from about one to several hundred liters per hectare. High strength compositions are primarily used as intermediates for further formulation. The formulations will typically contain effective amounts of active ingredient, diluent and surfactant within the following approximate ranges which add up 100 weight percent.

| | Weight Percent | | |
|---|---|---|---|
| | Active Ingredient | Diluent | Surfactant |
| Wettable Powders | 25-90 | 0-74 | 1-10 |
| Oil Suspensions, Emulsions, Solutions, (including Emulsifiable Concentrates) | 5-50 | 40-95 | 0-15 |
| Dusts | 1-25 | 70-99 | 0-5 |
| Granules, Baits and Pellets | 0.01-99 | 5-99.99 | 0-15 |
| High strength Compositions | 90-99 | 0-10 | 0-2 |

Typical solid diluents are described in Watkins, et al., *Handbook of Insecticide Dust Diluents and Carriers*, 2nd Ed., Dorland Books, Caldwell, New Jersey. Typical liquid diluents and solvents are described in Marsden, *Solvents Guide*, 2nd Ed., Interscience, New York, 1950. *McCutcheon's Detergents and Emulsifiers Annual*, Allured Publ. Corp., Ridgewood, New Jersey, as well as Sisely and Wood, *Encyclopedia of Surface Active Agents*, Chemical Publ. Co., Inc., New York, 1964, list surfactants and recommended uses. All formulations can contain minor amounts of additives to reduce foam, caking, corrosion, microbiological growth, etc.

Methods for formulating such compositions are well known. Solutions are prepared by simply mixing the ingredients. Fine solid compositions are made by blending and, usually, grinding as in a hammer mill or fluid energy mill. Water-dispersible granules can be produced be agglomerating a fine powder composition; see for example, Cross et al., *Pesticide Formulations*, Washington, D.C., 1988, pp 251-259. Suspensions are prepared by wet-milling; see, for example, U.S. 3,060,084. Granules and pellets can be made by spraying the active material upon preformed granular carriers or by agglomeration techniques. See Browning, "Agglomeration", *Chemical Engineering*, December 4, 1967, pp 147-148, *Perry's Chemical Engineer's Handbook*, 4th Ed., McGraw-Hill, New York, 1963, pp 8-57 and following, and WO 91/13546. Pellets can be prepared as described in U.S. 4,172,714. Water-dispersible and water-soluble granules can be prepared as taught in DE 3,246,493.

For further information regarding the art of formulation, see U.S. 3,235,361, Col. 6, line 16 through Col. 7, line 19 and Examples 10 through 41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, *Weed Control as a Science*, John Wiley and Sons, Inc., New York, 1961, pp 81-96; and Hance et al., *Weed Control Handbook*, 8th Ed., Blackwell Scientific Publications, Oxford, 1989.

In the following Examples, all percentages are by weight and all formulations are worked up in conventional ways. Compound numbers refer to Index Table A hereinafter.

### Example A

| Wettable Powder | |
|---|---|
| Compound 1 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0%. |

### Example B

| Granule | |
|---|---|
| Compound 1 | 10.0% |
| attapulgite granules (low volative matter, 0.71/0.30 mm; U.S.S. No. 25-50 sieves) | 90.0%. |

### Example C

| Extruded Pellet | |
|---|---|
| Compound 1 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0%. |

### Example D

| Emulsifiable Concentrate | |
|---|---|
| Compound 1 blend of oil soluble sulfonates | 20.0% |
| and polyoxyethylene ethers | 10.0% |
| isophorone | 70.0%. |

The compounds of this invention are useful as plant disease control agents. The present invention therefore further comprises a method for controlling plant diseases caused by fungal plant pathogens comprising applying to the plant or portion thereof to be protected, or to the plant seed or seedling to be protected, an effective amount of a compound of Formula I or a fungicidal composition containing said compound. The compounds and compositions of this invention provide control of diseases caused by a broad spectrum of fungal plant pathogens in the *Basidiomycete, Ascomycete, Oomycete and Deuteromycete* classes. They are effective in controlling a broad spectrum of plant diseases, particularly foliar pathogens of ornamental, vegetable, field, cereal, and fruit crops. These pathogens include *Plasmopara viticola*, *Phytophthora infestans*, *Peronospora tabacina*, *Pseudoperonospora cubensis*, *Pythium aphanidermatum*, *Alternaria brassicae*, *Septoria nodorum*, *Cercosporidium personatum*, *Cercospora arachidicola*, *Pseudocercosporella herpotrichoides*, *Cercospora beticola*, *Botrytis cinerea*, *Monilinia fructicola*, *Pyricularia oryzae*, *Podosphaera leucotricha*, *Venturia inaequalis*, *Erysiphe graminis*, *Uncinula necatur*, *Puccinia recondita*, *Puccinia graminis*, *Hemileia vastatrix*, *Puccinia striiformis*, *Puccinia arachidis*, *Rhizoctonia solani*, *Sphaerotheca fuliginea*, *Fusarium oxysporum*, *Verticillium dahliae*, *Pythium aphanidermatum*, *Phytophthora megasperma* and other generea and species closely related to these pathogens.

Compounds of this invention can also be mixed with one or more other insecticides, fungicides, nematocides, bactericides, acaricides, semiochemicals, repellants, attractants, pheromones, feeding stimulants or other biologically active compounds to form a multicomponent pesticide giving an even broader spectrum of agricultural protection. Examples of other agricultural protectants with which compounds of this invention can be formulated are: insecticides such as monocrotophos, carbofuran, tetrachlorvinphos, malathion, parathion-methyl, methomyl, chlordimeform, diazinon, deltamethrin, oxamyl, fenvalerate, esfenvalerate, permethrin, profenofos, sulprofos, triflumuron, diflubenzuron, methoprene, buprofezin, thiodicarb, acephate, azinphosmethyl, chlorpyrifos, dimethoate, fipronil, flufenprox, fonophos, isofenphos, methidathion, methamidophos, phosmet, phosphamidon, phosalone, pirimicarb, phorate, terbufos, trichlorfon, methoxychlor, bifenthrin, biphenate, cyfluthrin, fenpropathrin, fluvalinate, flucythrinate, tralomethrin, metaldehyde and rotenone; fungicides such as carbendazim, thiuram, dodine, maneb, chloroneb, benomyl, cymoxanil, fenpropidine, fenpropimorph, triadimefon, captan, thiophanate-methyl, thiabendazole, phosethyl-Al, chlorothalonil, dichloran, metalaxyl, captafol, iprodione, oxadixyl, vinclozolin, kasugamycin, myclobutanil, tebuconazole, difenoconazole, diniconazole, fluquinconazole, ipconazole, metconazole, penconazole, propiconazole, uniconzole, flutriafol, prochloraz, pyrifenox, fenarimol, triadimenol, diclobutrazol, copper oxychloride, furalaxyl, folpet, flusilazol, blasticidin S, diclomezine, edifenphos, isoprothiolane, iprobenfos, mepronil, neo-asozin, pencycuron, probenazole, pyroquilon, tricyclazole, validamycin, and flutolanil; nematocides such as aldoxycarb, fenamiphos and fosthietan; bactericides such as oxytetracyline, streptomycin and tribasic copper sulfate; acaricides such as binapacryl, oxythioquinox, chlorobenzilate, dicofol, dienochlor, cyhexatin, hexythiazox, amitraz, propargite, tebufenpyrad and fenbutatin oxide; and biological agents such as Bacillus thuringiensis, baculovirus and avermectin B.

In certain instances, combinations with other fungicides having a similiar spectrum of control but a different mode of action will be particularly advantageous for resistance management.

Plant disease control is ordinarily accomplished by applying an effective amount of a compound of this invention either pre- or post-infection, to the portion of the plant to be protected such as the roots, stems, foliage, fruit, seeds, tubers or bulbs, or to the media (soil or sand) in which the plants to be protected are growing. The compounds can also be applied to the seed to protect the seed and seedling.

Rates of application for these compounds can be influenced by many factors of the environment and should be determined under actual use conditions. Foliage can normally be protected when treated at a rate of from less than 1 g/ha to 5,000 g/ha of active ingredient. Seed and seedlings can normally be protected when seed is treated at a rate of from 0.1 to 10 g per kilogram of seed.

The following Tests demonstrate the control efficacy of compounds of this invention on specific pathogens. The pathogen control protection afforded by the compounds is not limited, however, to these species. See Index Table A for compound descriptions.

Test compounds were first dissolved in acetone in an amount equal to 3% of the final volume and then suspended at a concentration of 200 ppm in purified water containing 250 ppm of the surfactant Trem® 014 (polyhydric alcohol esters). The resulting test suspensions were then used in the following tests.

### TEST A

The test suspension was sprayed to the point of run-off on wheat seedlings. The following day the seedlings were inoculated with a spore dust of *Erysiphe graminis f. sp. tritici*, (the causal agent of wheat powdery mildew) and incubated in a growth chamber at 20°C for 7 days, after which disease ratings were made.

### TEST B

The test suspension was sprayed to the point of run-off on wheat seedlings. The following day the seedlings were inoculated with a spore suspension of *Puccinia recondita* (the causal agent of wheat leaf rust) and incubated in a saturated atmosphere at 20°C for 24 h, and then moved to a growth chamber at 20°C for 6 days, after which disease ratings were made.

### TEST C

The test suspension was sprayed to the point of run-off on rice seedlings. The following day the seedlings were inoculated with a spore suspension of *Pyricularia oryzae* (the causal agent of rice blast) and incubated in a saturated atmosphere at 27°C for 24 h, and then moved to a growth chamber at 30°C for 5 days, after which disease ratings were made.

### TEST D

The test suspension was sprayed to the point of run-off on tomato seedlings. The following day the seedlings were inoculated with a spore suspension of *Phytophthora infestans* (the causal agent of potato and tomato late blight) and incubated in a saturated atmosphere at 20°C for 24 h, and then moved to a growth chamber at 20°C for 5 days, after which disease ratings were made.

### TEST E

The test suspension was sprayed to the point of run-off on potato seedlings. The following day the seedlings were inoculated with a spore suspension of *Phytophthora infestans* (the causal agent of potato and tomato late blight) and incubated in a saturated atmosphere at 20°C for 24 h, and then moved to a growth chamber at 20°C for 5 days, after which disease ratings were made.

### TEST F

The test suspension was sprayed to the point of run-off on grape seedlings. The following day the seedlings were inoculated with a spore suspension of *Plasmopara viticola* (the causal agent of grape downy mildew) and incubated in a saturated atmosphere at 20°C for 24 h, moved to a growth chamber at 20°C for 6 days,and then incubated in a saturated atmosphere at 20°C for 24 h, after which disease ratings were made.

### TEST G

The test suspension was sprayed to the point of run-off on cucumber seedlings. The following day the seedlings were inoculated with a spore suspension of *Botrytis cinerea* (the causal agent of gray mold on many crops) and incubated in a saturated atmosphere at 20°C for 48 h, and moved to a growth chamber at 20°C for 5 days, after which disease ratings were made.

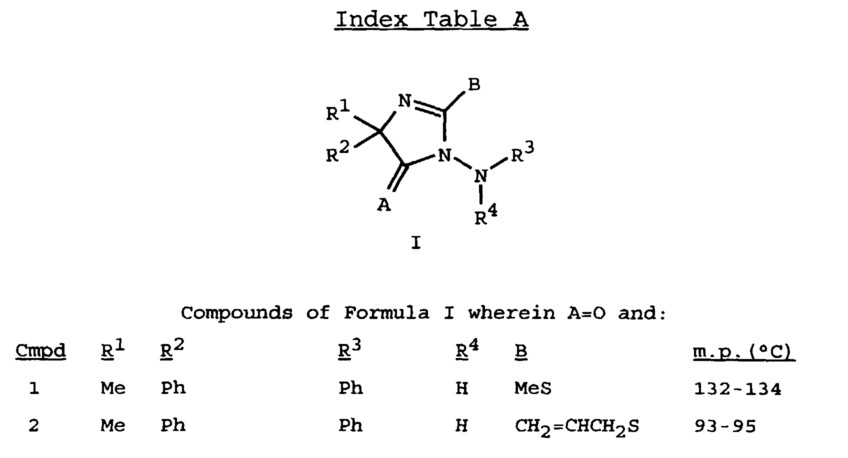

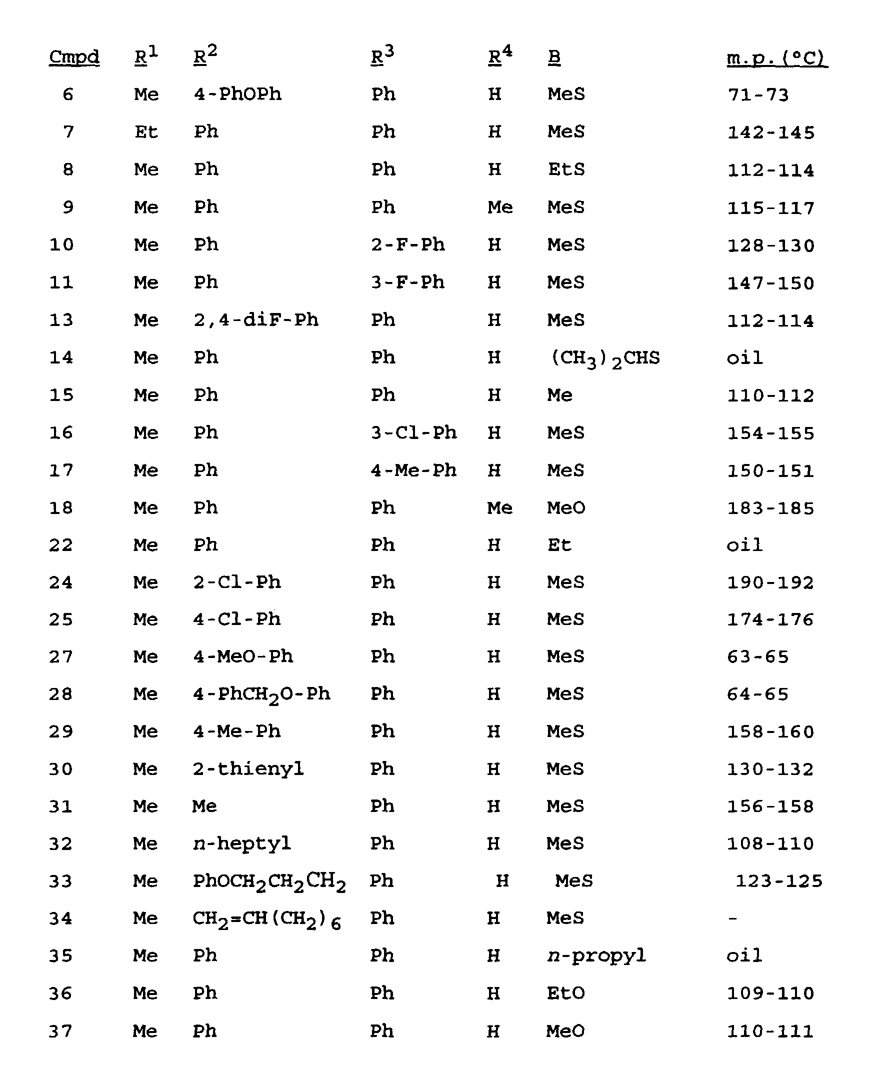

**Index Table B**

| Cmpd No. | ¹H NMR Data (CDCl₃, δ) |
|---|---|
| 14 | 1.48 (d, J=6.8 Hz, 3H), 1.52 (d, J=6.8 Hz, 3H), 1.76 (s, 3H), 4.03 (m, J=6.8 Hz, 1H), 6.10 (broad s, 1H), 6.93-7.62 (m, 10H) |
| 22 | 1.30 (t, J=7.4 Hz, 3H), 1.68 (s, 3H), 2.55 (q, J=7.4 Hz, 2H), 6.70 (s, 1H), 6.52-7.60 (m, 10H) |
| 34 | 0.95-1.30 (m, 8H), 1.23 (s, 3H), 1.50-2.00 (m, 4H), 2.45 (s, 3H), 4.78-5.12 (m, 2H), 5.50-5.92 (m, 1H), 6.40 (s, 1H), 6.61-7.30 (m, 5H) |
| 35 | 1.04 (t, J=7.6 Hz, 3H), 1.73 (s, 3H), 1.84 (m, 2H), 2.77 (q, J=7.6 Hz, 2H), 6.25 (s, 1H), 6.58-7.63 (m, 10H) |

Results for Tests A-G are given in Table C. In the table, a rating of 100 indicates 100% disease control and a rating of 0 indicates no disease control (relative to the controls). NT = not tested.

**TABLE C**

| Cmpd No. | Test A | Test B | Test C | Test D | Test E | Test F | Test G |
|---|---|---|---|---|---|---|---|
| 1 | 100** | 85* | 0 | NT | 98* | 100* | 0** |
| 2 | 0 | 26 | 0 | 60 | 50 | 100 | 0 |
| 6 | 0 | 85 | 0 | 24 | 82 | 100 | 14 |
| 8 | 0 | 40 | 0 | 67 | 37 | 96 | 0 |
| 9 | 0 | 100 | 0 | 89 | 86 | 100 | 0 |
| 10 | 0 | 97 | 0 | 95 | 100 | 100 | 5* |
| 11 | 0 | 100 | 0 | 99 | 100 | 100 | 0 |
| 13 | 0 | 100 | 53 | 99 | 100 | 100 | 0 |
| 14 | 49 | 0 | 0 | 0 | 0 | 91 | 0 |
| 15 | 49 | 95 | 0 | 97 | 95** | 100 | 70 |
| 16 | 49 | 100 | 0 | 97 | 92 | 100 | 0 |
| 17 | 49 | 100 | 0 | 99 | 95 | 100 | 0 |
| 18 | 49 | 100 | 74 | 97 | 99 | 100 | 0 |
| 24 | 77 | 53 | 0 | 99 | 100 | 100 | 0 |
| 25 | 86 | 100 | 0 | 99 | 100 | 100 | 0 |
| 27 | 55 | 100 | 18 | 99 | 100 | 100 | 0 |
| 28 | 26 | 100 | 99 | 73 | 92 | 99 | 0 |
| 29 | 55 | 100 | 18 | 99 | 100 | 100 | 11 |
| 30 | 30 | 100 | 0 | 97 | 100 | 100 | 0 |
| 31 | 47 | 80 | 31 | 44 | 68 | 97 | 0 |
| 32 | 61 | 96 | 0 | 93 | 100 | 100 | 62 |
| 33 | 31 | 91 | 0 | 99 | 100 | 100 | 0 |
| 35 | 0 | 1 | 0 | 63 | 29 | 75 | 63* |
| 36 | 40* | 0 | 0 | 0 | 0 | 56 | 0 |
| 37 | 13* | 97* | 0 | NT | 98* | 100* | NT |

| | | | | | | | |
|---|---|---|---|---|---|---|---|
| * The compound was sprayed at a concentration of 40 ppm | | | | | | | |
| ** The compound was sprayed at a concentration of 100 ppm | | | | | | | |

## Claims

1. A composition comprising:
(1) a compound of Formula I 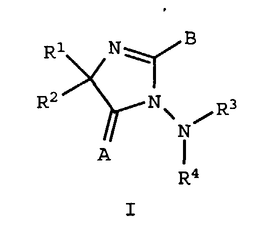 wherein
A is O or NH;
B is halogen; cyano; R²¹; OR²⁹; NR⁴⁹R⁶³; N=CR⁴⁵R⁴⁶; SR⁴⁷; or S(O)₂R⁴⁸;
R¹ is C₁-C₄ alkyl; C₁-C₄ haloalkyl; or vinyl;
R² is C₂-C₂₀ alkyl; C₂-C₂₀ alkoxyalkyl; C₃-C₈ alkyl substituted with phenoxy or phenylthio each optionally substituted with R³⁰; C₅-C₇ cycloalkyl; C₂-C₂₀ alkenyl; C₅-C₇ cycloalkenyl; phenyl optionally substituted with R⁵ and R⁷; 2-naphthalenyl; thienyl optionally substituted with R⁵ and R⁷; furyl optionally substituted with R⁷; or pyridyl optionally substituted with R⁵ and R⁷;
provided that when R² is phenyl and R⁵ is other than F, then R⁵ is attached to the para-position relative to the imidazolinone ring;
R³ is phenyl optionally substituted with R¹⁰;
R⁴ is H or methyl;
R⁵ is halogen; nitro; cyano; C₁-C₆ alkyl; C₅-C₆ cycloalkyl; C₁-C₆ haloalkyl; C₁-C₆ alkylthio; C₁-C₆ haloalkylthio; C₁-C₆ alkoxy; C₁-C₆ haloalkoxy; C₅-C₆ cycloalkyloxy; C₂-C₆ alkoxyalkyl; C₂-C₆ alkoxyalkoxy; C₃-C₆ alkenyl; C₃-C₆ haloalkenyl; C₃-C₆ alkenyloxy; C₃-C₆ alkynyl; C₃-C₆ haloalkynyl; C₃-C₆ alkynyloxy; C₁-C₆ alkylsulfonyl; C₁-C₆ haloalkylsulfonyl; phenyl or phenylthio each optionally substituted with R²⁴; phenylmethyl, phenoxymethyl, phenethyl, or styryl each optionally substituted with R²⁴ on the phenyl ring; phenoxy optionally substituted with R²⁷; benzyloxy optionally substituted with R³⁰ on the phenyl ring; -OC(=O)NHR²⁸; -C(=O)OR²⁸ or -OC(=O)R²⁸;
R⁷, R²⁴ and R²⁶ are independently 1-2 halogen; nitro; C₁-C₄ alkyl; trifluoromethyl; methylthio; or C₁-C₄ alkoxy;
R¹⁰ is 1-2 substituents selected from the group consisting of halogen, nitro, cyano, C₁-C₄ alkyl, trifluoromethyl, C₁-C₄ alkylthio, C₁-C₄ alkoxy and trifluoromethoxy;
R²¹ is C₁-C₄ alkyl optionally substituted with R⁵¹; C₂-C₄ alkenyl, C₂-C₈ alkynyl, or cyclopropyl each optionally substituted with 1-3 halogen; C(=N-V-R⁵³)H; C(=N-V-R⁵³)(C₁-C₄ alkyl); C(=O)OR⁵³; C(=O)SR⁵³; C(=S)SR⁵³; or C(=O)NR⁵³R⁵⁶;
V is O; NR⁵⁵; or a direct bond;
R²⁷ is 1-2 halogen; nitro; cyano; C₁-C₆ alkyl; C₁-C₆ haloalkyl; C₁-C₆ alkoxy; C₁-C₆ haloalkoxy; C₁-C₄ alkylsulfonyl; C₂-C₆ alkoxyalkyl; C₁-C₄ alkylthio; C₅-C₆ cycloalkyl; C₅-C₆ cycloalkyloxy; C₂-C₆ alkenyl; C₂-C₆ haloalkenyl; C₂-C₆ alkynyl; hydroxycarbonyl; C₂-C₄ alkoxycarbonyl; or phenoxy optionally substituted with R²⁴;
R²⁸ is C₁-C₈ alkyl; or phenyl or pyridyl each optionally substituted with R³⁰;
R²⁹ is C₁-C₄ alkyl optionally substituted with R⁴⁴; C₃-C₆ alkenyl, C₃-C₆ alkynyl, or cyclopropyl each optionally substituted with 1-3 halogen; C(=O)R⁵²; C(=NR⁵⁵)R⁵²; C(=O)OR⁵³; C(=O)NR⁵³R⁵⁶; N=CR⁶⁸R⁶⁷; or SO₂R⁵²;
R³⁰ is 1-2 substituents selected from the group consisting of halogen, nitro, cyano, C₁-C₄ alkyl, trifluoromethyl, C₁-C₄ alkoxy and trifluoromethoxy; or phenoxy optionally substituted with R²⁶;
R⁴⁴ is 1-3 halogen; C₁-C₄ alkoxy or C₁-C₄ haloalkoxy;
R⁴⁵ is H or C₁-C₄ alkyl;
R⁴⁶ is H; C₁-C₄ alkyl; OR⁶⁵; or SR⁶⁵;
R⁴⁷ is C₁-C₄ alkyl; C₃-C₄ alkenyl; C(=O)R⁵²; or C(=O)OR⁵³;
R⁴⁸ is C₁-C₄ alkyl;
R⁴⁹ is H; C₁-C₄ alkyl; C₃-C₄ alkenyl; or cyclopropyl;
R⁵¹ is 1-3 halogen or C₂-C₃ haloalkoxy;
R⁵² and R⁵³ are each independently H; C₁-C₄ alkyl; or C₃-C₄ alkenyl;
R⁵⁵ and R⁵⁶ are each independently H or C₁-C₄ alkyl;
R⁶³ is C₁-C₄ alkyl;
R⁶⁵ is C₁-C₄ alkyl or C₃-C₄ alkenyl;
R⁶⁷ is H or C₁-C₄ alkyl; and
R⁶⁸ is H; C₁-C₄ alkyl; C₁-C₄ haloalkyl; or C₂-C₄ alkenyl;
provided that the total number of carbons in R² is less than or equal to 20; and
provided that when R³ is a phenyl or heterocyclic ring disubstituted with two alkyl or alkoxy groups, or one alkyl and one alkoxy group, then at least one of the alkyl and alkoxy groups is methyl or methoxy;
and
(2) one or more compounds selected from monocrotophos, carbofuran, tetrachlorvinphos, malathion, parathion-methyl, methomyl, chlordimeform, diazinon, deltamethrin, oxamyl, fenvalerate, esfenvalerate, permethrin, profenofos, sulprofos, triflumuron, diflubenzuron, methoprene, buprofezin, thiodicarb, acephate, azinphosmethyl, chlorpyrifos, dimethoate, fipronil, flufenprox, fonophos, isofenphos, methidathion, methamidophos, phosmet, phosphamidon, phosalone, pirimicarb, phorate, terbufos, trichlorfon, methoxychlor, bifenthrin, biphenate, cyfluthrin, fenpropathrin, fluvalinate, flucythrinate, tralomethrin, metaldehyde, rotenone, carbendazim, thiuram, dodine, maneb, chloroneb, benomyl, cymoxanil, fenpropidine, fenpropimorph, triadimefon, captan, thiophanate-methyl, thiabendazole, phosethyl-Al, chlorothalonil, dichloran, metalaxyl, captafol, iprodione, oxadixyl, vinclozolin, kasugamycin, myclobutanil, tebuconazole, difenoconazole, diniconazole, fluquinconazole, ipconazole, metconazole, penconazole, propiconazole, uniconzole, flutriafol, prochloraz, pyrifenox, fenarimol, triadimenol, diclobutrazol, copper oxychloride, furalaxyl, folpet, flusilazol, blasticidin S, diclomezine, edifenphos, isoprothiolane, iprobenfos, mepronil, neo-asozin, pencycuron, probenazole, pyroquilon, tricyclazole, validamycin, flutolanil, aldoxycarb, fenamiphos and fosthietan, oxytetracyline, streptomycin tribasic copper sulfate, binapacryl, oxythioquinox, chlorobenzilate, dicofol, dienochlor, cyhexatin, hexythiazox, amitraz, propargite, tebufenpyrad, fenbutatin oxide, Bacillus thuringiensis, baculovirus and avermectin B.

2. A composition as claimed in Claim 1 further comprising at least one of (a) a surfactant, (b) an organic solvent, and (c) at least one solid or liquid diluent.

3. A composition comprising:
(1) a compound of Formula I as defined in Claim 1 and
(2) at least one insecticide, nematocide, bactericide, acaricide, semiochemical, repellent, attractant, pheromone or feeding stimulant.

4. A composition comprising:
(1) a compound of Formula I as defined in Claim 1; and
(2) at least one other fungicide having a different mode of action from the compound of Formula I.

5. A composition as claimed in any one of Claims 1 to 4 wherein in the compound of Formula I,
B is halogen; cyano; C₁-C₄ alkyl, or C₁-C₄ alkoxy each optimally substituted with halogen; C₁-C₄ alkylthio; N=CR⁴⁵R⁴⁶; NR⁴⁹R⁶³; or S(O)₂(C₁-C₄ alkyl);
R¹ is methyl or halomethyl;
R² is C₂-C₁₂ alkyl; C₃-C₈ alkyl substituted wit phenoxy optionally substituted with R³⁰; phenyl optionally substituted with R⁵ and R⁷ thienyl optionally substituted with R⁷; or pyridyl optionally substituted with R⁵ and R⁷;
R³ is phenyl optionally substituted with F, Cl, or methyl;
R⁴ is H;
R⁵ is halogen; nitro; C₁-C₆ alkyl; C₁-C₃ haloalkyl; metylthio; C₁-C₆ alkoxy; C₁-C₂ haloalkoxy; C₅-C₆ cycloalkyloxy; phenoxy optionally substituted with R²⁷; phenylthio substituted with R²⁴; phenoxymethyl optionally substituted with R²⁴ on the phenyl ring; benzyloxy optionally substituted with R³⁰ on the phenyl ring; or -OC(=O)R²⁸;
R⁷ and R²⁴ are independently F; C₁-C₂ alkyl; methylthio; or C₁-C₂ alkoxy;
R²⁷ is 1-2 halogen; cyano; C₁-C₄ alkyl; trifluoromethyl; C₁₋C₄ alkoxy; C₁-C₄ haloalkoxy; C₁-C₄ alkylthio; C₅-C₆ cycloalkyloxy; or allyl;
R³⁰ is 1-2 halogen; cyano; C₁-C₄ alkyl; trifluoromethyl; C₁₋C₄ alkoxy; or trifluoromethoxy;
R⁴⁶ is H or C₁-C₄ alkyl; and
R⁴⁹ and R⁶³ are each independently C₁-C₂ alkyl.

6. A composition as claimed in any one of Claims 1 to 4 wherein in the compound of Formula I,
B is F; Cl; cyano; C₁-C₂ alkyl; C₁-C₂ alkylthio; C₁-C₂ alkoxy; N=CR⁴⁵R⁴⁶; NMe₂; or S(O)₂(C₁-C₂ alkyl);
R¹ is methyl;
R² is C₂-C₁₂ alkyl; phenyl optionally substituted with R⁵ and R⁷; or thienyl optionally substituted with R⁵ and R⁷; and
R⁵ is F; Cl; Br; C₁-C₆ alkyl; trifluoromethyl; C₁-C₆ alkoxy; trifluoromethoxy; 2,2,2-trifluoroethoxy; C₅-C₆ cycloalkyloxy; metylthio; phenoxy optionally substituted with R²⁷; phenylthio optionally substituted with R²⁴; benzyloxy optionally substituted with R³⁰ on the phenyl ring; or -OC(=O)R²⁸.

7. A composition as claimed in any one of Claims 1 to 6 wherein R¹ and R² are different in the compound of Formula I, and the compound of Formula I is in enantiomerically pure form.

8. A composition as claimed in any one of Claims 1 to 7 where the compound of Formula I is 3,5-dihydro-5-methyl-2-(methylthio)-5-phenyl-3-(phenylamino)-4*H*-imidazol-4-one.

9. A method of controlling plant diseases caused by fungi selected from the group *Peronospora tabacina*, *Pseudoperonospora cubensis*, *Pythium aphanidermatum*, *Alternaria brassicae*, *Cercosporidium personatum*, *Cercospora arachidicola*, *Cercospora beticola*, *Botrytis cinerea*, *Monilinia fructicola*, *Pyricularia oryzae*, *Podosphaera leucotricha*, *Venturia inaequalis*, *Erysiphe graminis*, *Uncinula necatur*, *Puccinia graminis*, *Hemileia vastatrix*, *Puccinia striiformis*, *Puccinia arachidis*, *Sphaerotheca fuliginea*, *Verticillium dahliae*, *Pythium aphanidermatum*, and *Phytophthora megasperma* comprising applying to the plant or portion thereof to be protected an effective amount of a fungicidal composition comprising a compound of Formula I as defined in Claim 1.

10. A method as claimed in Claim 9 wherein the fungi are selected from the group *Erysiphe graminis*, *Pyricularia oryzae* and *Botrytis cinerea* are controlled.

11. A method as claimed in Claim 9 or Claim 10 wherein in the compounds of Formula I, R¹ and R² are different and the compound is in enantiomerically pure form.

12. A method as claimed in any one of Claims 9 to 11 wherein the compound of Formula I is 3,5-dihydro-5-methyl-2-(methylthio)-5-phenyl-3-(phenylamino)-4*H*-imidazol-4-one.

13. A compound of Formula I as defined in Claim 1, 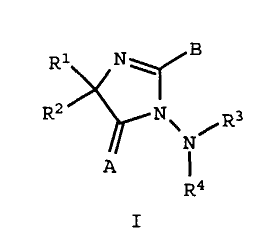 with the proviso that when A denotes O, and either (a) B denotes OR²⁹ and R²⁹ denotes C₁-C₄ alkyl optionally substituted with R⁴⁴, C₃-C₆ alkenyl or C₃-C₆ alkynyl, or (b) B denotes SR⁴⁷ and R⁴⁷ denotes C₁-C₄ alkyl or C₃-C₄ alkenyl,
then R¹ and R² are different, and the compound is not in racemic form.

14. A compound as claimed in Claim 13 wherein R¹ and R² are different which has the stereochemistry

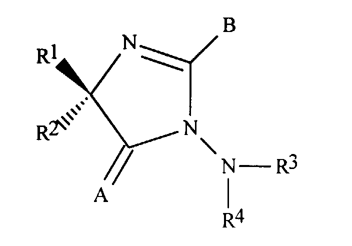

15. A compound as claimed in Claim 13 or Claim 14 which is 3,5-dihydro-5-methyl-2-(methylthio)-5-phenyl-3 -(phenylamino)-4*H*-imidazol-4-one.

16. A compound of Formula I as defined in Claim I with the proviso that when A denotes O and either (a) B denotes OR²⁹, then R²⁹ does not denote C₁-C₄ optionally substituted with R⁴⁴, C₃-C₆ alkenyl or C₃-C₆ alkynyl; or (b) B denotes SR⁴⁷, then R⁴⁷ does not denote C₁-C₄ alkyl, or C₃-C₄ alkenyl.

17. A compound as claimed in Claim 16 wherein
B is halogen; cyano; C₁-C₄ alkyl, or C₁-C₄ alkoxy each optionally substituted with halogen; C₁-C₄ alkylthio; N=CR⁴⁵R⁴⁶; NR⁴⁹R⁶³; or S(O)₂(C₁-C₄ alkyl);
R¹ is methyl or halomethyl;
R² is C₂-C₁₂ alkyl; C₃-C₈ alkyl substituted with phenoxy optionally substituted with R³⁰; phenyl optionally substituted with R⁵ and R⁷ thienyl optionally substituted with R⁷; or pyridyl optionally substituted with R⁵ and R⁷;
R³ is phenyl optionally substituted with F; Cl; or methyl;
R⁴ is H;
R⁵ is halogen; nitro; C₁-C₆ alkyl; C₁-C₃ haloalkyl; methylthio; C₁-C₆ alkoxy; C₁-C₂ haloalkoxy; C₅-C₆ cycloalkyloxy; phenoxy optionally substituted with R; phenylthio substituted with R²⁴; phenoxymethyl optionally substituted with R²⁴ on the phenyl ring; benzyloxy optionally substituted with R³⁰ on the phenyl ring; or -OC(=O)R²⁸;
R⁷ and R²⁴ are independently F; C₁-C₂ alkyl; methylthio; or C₁-C₂ alkoxy;
R²⁷ is 1-2 halogen; cyano; C₁-C₄ alkyl; trifluoromethyl; C₁₋C₄ alkoxy; C₁-C₄ haloalkoxy; C₁-C₄ alkylthio; C₅-C₆ cycloalkyloxy; or allyl;
R³⁰ is 1-2 halogen; cyano; C₁-C₄ alkyl; trifluoromethyl; C₁₋C₄ alkoxy; or trifluoromethoxy;
R⁴⁶ is H or C₁-C₄ alkyl;
R⁴⁹ and R⁶³ are each independently C₁-C₂ alkyl.

18. A compound as claimed in Claim 16 wherein
B is F; Cl; cyano; C₁-C₂ alkyl; C₁-C₂ alkylthio; C₁-C₂ alkoxy; N=CR⁴⁵R⁴⁶; NMe₂; or S(O)₂(C₁-C₂ alkyl);
R¹ is methyl;
R² is C₂-C₁₂ alkyl; phenyl optionally substituted with R⁵ and R⁷; or thienyl optionally substituted with R⁵ and R⁷; and
R⁵ is F; Cl; Br; C₁-C₆ alkyl; trifluoromethyl; C₁-C₆ alkoxy; trifluoromethoxy; 2,2,2-trifluoroethoxy; C₅-C₆ cycloalkyloxy; methylthio; phenoxy optionally substituted with R²⁷; phenylthio optionally substituted with R²⁴; benzyloxy optionally substituted with R³⁰ on the phenyl ring; or -OC(=O)R²⁸.

19. A composition comprising a compound of Formula I as claimed in any of Claims 13 to 18 and at least one of (a) a surfactant, (b) an organic solvent, and (c) at least one solid or liquid diluent.

20. A method of controlling plant diseases comprising applying to the plant or portion thereof to be protected, or to the plant seed or seedling to be protected, an effective amount of a fungicidal composition as claimed in any of Claims 1 to 8 or 19.

## Patentansprüche

1. Zusammensetzung, umfassend:
(1) eine Verbindung der Formel I 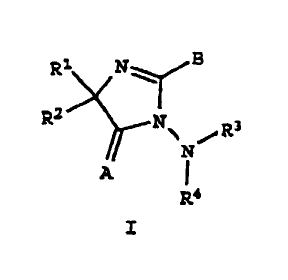 worin
A O oder NH ist;
B für Halogen; Cyano; R²¹; OR²⁹; NR⁴⁹R⁶³; N=CR⁴⁵R⁴⁶; SR⁴⁷ oder S(O)₂R⁴⁸ steht;
R¹ für C₁- bis C₄-Alkyl; C₁- bis C₄-Haloalkyl oder Vinyl steht;
R² für C₂- bis C₂₀-Alkyl; C₂- bis C₂₀-Alkoxyalkyl; C₃- bis C₈-Alkyl, substituiert mit Phenoxy oder Phenylthio, von denen jedes optional mit R³⁰ substituiert ist; C₅- bis C₇-Cycloalkyl; C₂- bis C₂₀-Alkenyl; C₅- bis C₇-Cycloalkenyl; Phenyl, das optional mit R⁵ und R⁷ substituiert ist; 2-Naphthalenyl; Thienyl, das optional mit R⁵ und R⁷ substituiert ist; Furyl, das optional mit R⁷ substituiert ist, oder Pyridyl, das optional mit R⁵ und R⁷ substituiert ist, steht;
unter der Voraussetzung, daß, wenn R² Phenyl ist und R⁵ von F verschieden ist, dann R⁵ in para-Stellung relativ zu dem Imidazolinonring gebunden ist;
R³ für Phenyl, das optional mit R¹⁰ substituiert ist, steht;
R⁴ für H oder Methyl steht;
R⁵ für Halogen; Nitro; Cyano; C₁- bis C₆-Alkyl; C₅- bis C₆-Cycloalkyl, C₁- bis C₆-Haloalkyl; C₁- bis C₆-Alkylthio; C₁- bis C₆-Haloalkylthio; C₁- bis C₆-Alkoxy; C₁- bis C₆-Haloalkoxy; C₅- bis C₆-Cycloalkyloxy; C₂- bis C₆-Alkoxyalkyl, C₂- bis C₆-Alkoxyalkoxy; C₃- bis C₆-Alkenyl; C₃- bis C₆-Haloalkenyl; C₃- bis C₆-Alkenyloxy; C₃- bis C₆-Alkynyl; C₃- bis C₆-Haloalkynyl; C₃- bis C₆-Alkynyloxy; C₁- bis C₆-Alkylsulfonyl; C₁- bis C₆-Haloalkylsulfonyl; Phenyl oder Phenylthio, von denen jedes optional mit R²⁴ substituiert ist; Phenylmethyl; Phenoxymethyl, Phenethyl oder Styryl, von denen jedes optional mit R²⁴ auf dem Phenylring substituiert ist; Phenoxy, das optional mit R²⁷ substituiert ist; Benzyloxy, das optional mit R³⁰ auf dem Phenylring substituiert ist; ―OC(=O)NHR²⁸, ―C(=O)OR²⁸ oder ―OC(=O)R²⁸ steht;
R⁷, R²⁴ und R²⁶ unabhängig voneinander für 1-2 Halogene; Nitro; C₁- bis C₄-Alkyl; Trifluormethyl; Methylthio oder C₁- bis C₄-Alkoxy stehen;
R¹⁰ für 1-2-Substituenten, ausgewählt aus der Gruppe bestehend aus Halogen, Nitro, Cyano, C₁- bis C₄-Alkyl, Trifluormethyl, C₁- bis C₄-Alkylthio, C₂- bis C₄-Alkoxy und Trifluormethoxy, steht;
R²¹ für C₁-C₄-Alkyl, optional substituiert mit R⁵¹; C₂- bis C₄-Alkenyl, C₂- bis C₈-Alkynyl oder Cyclopropyl, von denen jedes optional mit 1-3 Halogenen substituiert ist; C(=N-V-R⁵³)H; C(=N-V-R⁵³)(C₁- bis C₄-Alkyl); C(=O)OR⁵³; C(=O)SR⁵³; C(=S)SR⁵³ oder C(O=)NR⁵³R⁵⁶ steht;
V für O; NR⁵⁵ oder eine direkte Bindung steht;
R²⁷ für 1-2 Halogene; Nitro; Cyano; C₁- bis C₆-Alkyl; C₁- bis C₆-Haloalkyl; C₁ bis C₆-Alkoxy; C₁- bis C₆-Haloalkoxy; C₁- bis C₄-Alkylsulfonyl; C₂- bis C₆-Alkoxyalkyl; C₁- bis C₄-Alkylthio; C₅- bis C₆-Cycloalkyl; C₅- bis C₆-Cycloalkyloxy; C₂- bis C₆-Alkenyl; C₂- bis C₆-Haloalkenyl; C₂- bis C₆-Alkynyl; Hydroxycarbonyl; C₂- bis C₄-Alkoxycarbonyl oder Phenoxy, das optional mit R²⁴ substituiert ist, steht;
R²⁸ für C₁- bis C₈-Alkyl; oder Phenyl oder Pyridyl, von denen jedes optional mit R³⁰ substituiert ist, steht;
R²⁹ für C₁- bis C₄-Alkyl, das optional mit R⁴⁴ substituiert ist; C₃- bis C₆-Alkenyl, C₃- bis C₆-Alkynyl oder Cyclopropyl, von denen jedes optional mit 1-3 Halogenen substituiert ist; C(=O)R⁵²; C(=NR⁵⁵)R⁵²; C(=O)OR⁵³; C(=O)NR⁵³R⁵⁶; N=CR⁶⁸R⁶⁷ oder SO₂R⁵² steht;
R³⁰ für 1-2 Substituenten, die ausgewählt sind aus der Gruppe bestehend aus Halogen, Nitro, Cyano, C₁- bis C₄-Alkyl, Trifluormethyl, C₁- bis C₄-Alkoxy und Trifluormethoxy; oder Phenoxy, das optional mit R²⁶ substituiert ist, steht;
R⁴⁴ für 1-3 Halogene; C₁- bis C₄-Alkoxy oder C₁- bis C₄-Haloalkoxy steht;
R⁴⁵ für H oder C₁- bis C₄-Alkyl steht,
R⁴⁶ für H; C₁- bis C₄-Alkyl; OR⁶⁵ oder SR⁶⁵ steht;
R⁴⁷ für C₁- bis C₄-Alkyl; C₃- bis C₄-Alkenyl; C(=O)R⁵² oder C(=O)OR⁵³ steht;
R⁴⁸ für C₁- bis C₄-Alkyl steht;
R⁴⁹ für H; C₁- bis C₄-Alkyl; C₃- bis C₄-Alkenyl oder Cyclopropyl steht;
R⁵¹ für 1-3-Halogen oder C₂- bis C₃-Haloalkoxy steht;
R⁵² und R⁵³ jeweils unabhängig voneinander für H; C₁- bis C₄-Alkyl oder C₃- bis C₄-Alkenyl stehen;
R⁵⁵ und R⁵⁶ jeweils unabhängig voneinander für H oder C₁- bis C₄-Alkyl stehen;
R⁶³ für C₁- bis C₄-Alkyl steht;
R⁶⁵ für C₁- bis C₄-Alkyl oder C₃- bis C₄-Alkenyl steht,
R⁶⁷ für H oder C₁- bis C₄-Alkyl steht und
R⁶⁸ für H; C₁- bis C₄-Alkyl; C₁- bis C₄-Haloalkyl oder C₁- bis C₄-Alkenyl steht;
unter der Voraussetzung, daß die Gesamtzahl der Kohlenstoffatome in R² kleiner oder gleich 20 ist, und unter der Voraussetzung, daß, wenn R³ ein Phenyl oder heterocyclischer Ring ist, der mit zwei Alkyl- oder Alkoxygruppen oder einer Alkyl- und einer Alkoxygruppe substituiert ist, dann mindestens eine der Alkyl- und Alkoxygruppen Methyl oder Methoxy ist; und
(2) eine oder mehrere Verbindungen, ausgewählt aus Monocrotophos, Carbofuran, Tetrachlorvinphos, Malathion, Parathionmethyl, Methomyl, Chlordimeform, Diazinon, Deltamethrin, Oxamyl, Fenvalerat, Esfenvalerat, Permethrin, Profenofos, Sulprofos, Triflumuron, Diflubenzuron, Methopren, Buprofezin, Thiodicarb, Acephat, Azinphosmethyl, Chlorpyrifos, Dimethoat, Fipronil, Flufenprox, Fonophos, Isofenphos, Methidathion, Methamidophos, Phosmet, Phosphamidon Phosalon, Pirimicarb, Phorat, Terbufos, Trichlorfon, Methoxychlor, Bifenthrin, Biphenat, Cyfluthrin, Fenpropathrin, Fluvalinat, Flucythrinat, Tralomethrin, Metaldehyd, Rotenon, Carbendazim, Thiuram, Dodin, Maneb, Chloroneb, Benomyl, Cymoxanil, Fenpropidin, Fenpropimorph, Triadimefon, Captan, Thiophanatmethyl, Thiabendazol, Phosethyl-Al, Chlorthalonil, Dichloran, Metalaxyl, Captafol, Iprodion, Oxadixyl, Vinclozolin, Kasugamycin, Myclobutanil, Tebuconazol, Difenoconazol, Diniconazol, Fluquinconazol, Ipconazol, Metconazol, Penconazol, Propiconazol, Uniconzol, Flutriafol, Prochloraz, Pyrifenox, Fenarimol, Triadimenol, Diclobutrazol, Kupferoxychlorid, Furalaxyl, Folpet, Flusilazol, Blasticidin S, Diclomezin, Edifenphos, Isoprothiolan, Iprobenfos, Mepronil, Neo-Asozin, Pencycuron, Probenazol, Pyroquilon, Tricyclazol, Validamycin, Flutolanil, Aldoxycarb, Fenamiphos und Fosthietan, Oxytetracyclin, dreibasisches Streptomycin-Kupfersulfat, Binapacryl, Oxythioqinox, Chlorobenzilat, Dicofol, Dienochlor, Cyhexatin, Hexythiazox, Amitraz, Propargit, Tebufenpyrad, Fenbutatinoxid, Bacillus thuringiensis, Baculovirus und Avermectin B.

2. Zusammensetzung nach Anspruch 1, weiterhin umfassend mindestens eines von (a) einem Tensid, (b) einem organischen Lösungsmittel und (c) mindestens einem festen oder flüssiges Verdünnungsmittel.

3. Zusammensetzung, umfassend:
(1) eine Verbindung der Formel I, wie in Anspruch 1 definiert, und
(2) mindestens ein(en) Insektizid, Nematozid, Bakterizid, Acarizid, Geruchsstoff, Repellent, Lockstoff, Pheromon oder Futteraufnahmestimulans.

4. Zusammensetzung, umfassend:
(1) eine Verbindung der Formel I, wie in Anspruch 1 definiert, und
(2) mindestens eines anderes Fungizid, das eine andere Wirkungsweise aufweist als die Verbindung der Formel I.

5. Zusammensetzung nach einem der Ansprüche 1 bis 4, wobei in der Verbindung der Formel I
B für Halogen; Cyano; C₁- bis C₄-Alkyl oder C₁- bis C₄-Alkoxy, von denen jedes optional mit Halogen substituiert ist; C₁- bis C₄-Alkylthio; N=CR⁴⁵R⁴⁶; NR⁴⁹R⁶³ oder S(O)₂(C₁- bis C₄-Alkyl) steht;
R¹ für Methyl oder Halomethyl steht;
R² für C₂- bis C₁₂-Alkyl; C₃- bis C₈-Alkyl, das mit optional mit R³⁰ substituiertem Phenoxy substituiert ist; Phenyl, das optional mit R⁵ und R⁷ substituiert ist; Thienyl, das optional mit R⁷ substituiert ist, oder Pyridyl, das optional mit R⁵ und R⁷ substituiert ist, steht;
R³ für Phenyl, das optional mit F, Cl oder Methyl substituiert ist, steht;
R⁴ für H steht;
R⁵ für Halogen; Nitro; C₁- bis C₆-Alkyl; C₁- bis C₃-Haloalkyl; Methylthio; C₁- bis C₆-Alkoxy; C₁- bis C₂-Haloalkoxy; C₅- bis C₆-Cycloalkyloxy; Phenoxy, das optional mit R²⁷ substituiert ist; Phenylthio, das mit R²⁴ substituiert ist; Phenoxymethyl, das optional mit R²⁴ auf dem Phenylring substituiert ist; Benzyloxy, das optional mit R³⁰ auf dem Phenylring substituiert ist, oder ―OC(=O)R²⁸ steht.
R⁷ und R²⁴ unabhängig voneinander für F; C₁- bis C₂-Alkyl; Methylthio oder C₁-bis C₂-Alkoxy stehen;
R²⁷ für 1-2 Halogene; Cyano; C₁- bis C₄-Alkyl; Trifluormethyl; C₁- bis C₄-Alkoxy; C₁- bis C₄-Haloalkoxy; C₁- bis C₄-Alkylthio; C₅- bis C₆-Cycloalkyloxy oder Allyl steht;
R³⁰ für 1-2 Halogene; Cyano; C₁- bis C₄-Alkyl; Trifluormethyl; C₁- bis C₄-Alkoxy oder Trifluormethoxy steht;
R⁴⁶ für H oder C₁ bis C₄-Alkyl steht,
R⁴⁹ und R⁶³ jeweils unabhängig voneinander für C₁- bis C₂-Alkyl stehen.

6. Zusammensetzung nach einem der Ansprüche 1 bis 4, wobei in der Verbindung der Formel I
B für F; Cl; Cyano; C₁- bis C₂-Alkyl; C₁- bis C₂-Alkylthio; C₁- bis C₂-Alkoxy; N=CR⁴⁵R⁴⁶; NMe₂, oder S(O)₂(C₁- bis C₂-Alkyl) steht;
R¹ für Methyl steht;
R² für C₂- bis C₁₂-Alkyl; Phenyl, das optional mit R⁵ oder R⁷ substituiert ist, oder Thienyl, das optional mit R⁵ und R⁷ substituiert ist, steht und
R⁵ für F; Cl; Br; C₁- bis C₆-Alkyl; Trifluormethyl; C₁- bis C₆-Alkoxy; Trifluormethoxy; 2,2,2-Trifluorethoxy; C₅- bis C₆-Cycloalkyloxy; Methylthio; Phenoxy, das optional mit R²⁷ substituiert ist; Phenylthio, das optional mit R²⁴ substituiert ist; Benzyloxy, das optional mit R³⁰ auf dem Phenylring substituiert ist, oder ―OC(=O)R²⁸ steht.

7. Zusammensetzung nach einem der Ansprüche 1 bis 6, wobei R¹ und R² in der Verbindung der Formel I verschieden sind und die Verbindung der Formel I in optisch reiner Form vorliegt.

8. Zusammensetzung nach einem der Ansprüche 1 bis 7, wobei es sich bei der Verbindung der Formel I um 3,5-Dihydro-5-methyl-2-(methylthio)-5-phenyl-3-(phenylamino)-4*H*-imidazol-4-on handelt.

9. Verfahren zum Bekämpfen von Pflanzenkrankheiten, die durch Pilze, ausgewählt aus der Gruppe *Peronospora tabacina, Pseudoperonospora cubensis*, *Pythium* *aphanidermatum, Alternaria brassicae, Cercosporidium personatum, Cercospora arachidicola, Cercospora beticola, Botrytis cinerea, Monilinia fructicola, Pyricularia oryzae, Podosphaera leucotricha, Venturia inaequalis, Erysiphe graminis, Uncinula necatur, Puccinia graminis, Hemileia vastatrix, Puccinia striiformis, Puccinia arachidis, Sphaerotheca fuliginea, Verticillium dahliae, Pythium aphanidermatum* und *Phytophtora megasperma* hervorgerufen werden, umfassend das Aufbringen einer wirksamen Menge einer fungiziden Zusammensetzung, die eine Verbindung der Formel I wie in Anspruch 1 definiert umfaßt, auf die zu schützende Pflanze oder einen Teile von dieser.

10. Verfahren nach Anspruch 9, wobei die Pilze, die aus der Gruppe *Erysiphe graminis, Pyricularia oryzae* und *Botrytis cinerea* ausgewählt sind, bekämpft werden.

11. Verfahren nach einem der Ansprüche 9 oder 10, wobei in den Verbindungen der Formel I R¹ und R² verschieden sind und die Verbindung in optisch reiner Form vorliegt.

12. Verfahren nach einem der Ansprüche 9 bis 11, wobei es sich bei der Verbindung der Formel I um 3,5-Dihydro-5-methyl-2-(methylthio)-5-phenyl-3-(phenylamino)-4*H*-imidazol-4-on handelt.

13. Verbindung der Formel I wie in Anspruch I definiert, 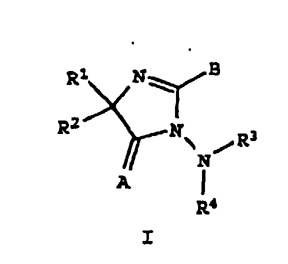 mit der Maßgabe, daß, wenn A O bedeutet und (a) B OR²⁹ bedeutet und R²⁹ C₁- bis C₄-Alkyl, das optional mit R⁴⁴ substituiert ist, C₃- bis C₆-Alkenyl oder C₃- bis C₆-Alkynyl bedeutet, oder (b) B SR⁴⁷ bedeutet und R⁴⁷ C₁- bis C₄-Alkyl oder C₃- bis C₄-Alkenyl bedeutet, dann R¹ und R² verschieden sind, und die Verbindung nicht in racemischer Form vorliegt.

14. Verbindung nach Anspruch 13, worin R¹ und R² verschieden sind, welche die Stereochemie 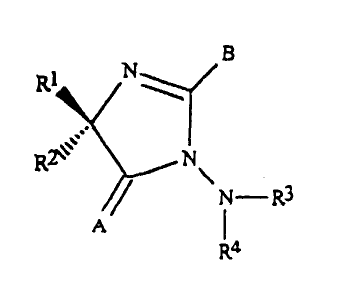 aufweist.

15. Verbindung nach einem der Ansprüche 13 oder 14, bei der es sich um 3,5-Dihydro-5-methyl-2-(methylthio)-5-phenyl-3-(phenylamino)-4*H*-imidazol-4-on handelt.

16. Verbindung der Formel I wie in Anspruch 1 definiert, mit der Maßgabe, daß, wenn A O bedeutet und (a) B OR²⁹ bedeutet, dann R²⁹ nicht C₁ bis C₄, das optional mit R⁴⁴ substituiert ist, C₃- bis C₆-Alkenyl oder C₃- bis C₆-Alkynyl bedeutet; oder (b) B SR⁴⁷ bedeutet, dann R⁴⁷ nicht C₁- bis C₄-Alkyl oder C₃- bis C₄-Alkenyl bedeutet.

17. Verbindung nach Anspruch 16, worin
B für Halogen; Cyano; C₁- bis C₄-Alkyl oder C₁- bis C₄-Alkoxy, von denen jedes optional mit Halogen substituiert ist; C₁- bis C₄-Alkylthio; N=CR⁴⁵R⁴⁶; NR⁴⁹R⁶³ oder S(O)₂(C₁- bis C₄-Alkyl) steht;
R^{¹} für Methyl oder Halomethyl steht;
R² für C₂- bis C₁₂-Alkyl; C₃- bis C₈-Alkyl, das mit optional mit R³⁰ substituiertem Phenoxy substituiert ist; Phenyl, das optional mit R⁵ und R⁷ substituiert ist; Thienyl, das optional mit R⁷ substituiert ist, oder Pyridyl, das optional mit R⁵ und R⁷ substituiert ist, steht;
R³ für Phenyl, das optional mit F; Cl oder Methyl substituiert ist, steht;
R⁴ für H steht;
R⁵ für Halogen; Nitro; C₁- bis C₆-Alkyl; C₁- bis C₃-Haloalkyl; Methylthio; C₁- bis C₆-Alkoxy; C₁- bis C₂-Haloalkoxy; C₅- bis C₆-Cycloalkyloxy; Phenoxy, das optional mit R²⁷ substituiert ist; Phenylthio, das mit R²⁴ substituiert ist; Phenoxymethyl, das optional mit R²⁴ auf dem Phenylring substituiert ist; Benzyloxy, das optional mit R³⁰ auf dem Phenylring substituiert ist, oder ―OC(O)R²⁸ steht;
R⁷ und R²⁴ unabhängig voneinander für F; C₁- bis C₂-Alkyl; Methylthio oder C₁- bis C₂-Alkoxy stehen;
R²⁷ für 1-2 Halogene; Cyano; C₁- bis C₄-Alkyl; Trifluormethyl; C₁- bis C₄-Alkoxy, C₁- bis C₄-Haloalkoxy; C₁- bis C₄-Alkylthio; C₅- bis C₆-Cycloalkyloxy oder Allyl steht;
R³⁰ für 1-2 Halogene; Cyano; C₁- bis C₄-Alkyl; Trifluormethyl; C₁- bis C₄-Alkoxy oder Trifluormethoxy steht;
R⁴⁶ für H oder C₁- bis C₄-Alkyl steht;
R⁴⁹ und R⁶³ jeweils unabhängig voneinander für C₁- bis C₂-Alkyl stehen.

18. Verbindung nach Anspruch 16, wobei
B für F; Cl; Cyano; C₁- bis C₂-Alkyl; C₁- bis C₂-Alkylthio; C₁- bis C₂-Alkoxy; N=CR⁴⁵R⁴⁶; NMe² oder S(O)₂(C₁- bis C₂-Alkyl) steht;
R¹ für Methyl steht;
R² für C₂- bis C₁₂-Alkyl; Phenyl, das optional mit R⁵ und R⁷ substituiert ist, oder Thienyl, das optional mit R⁵ und R⁷ substituiert ist, steht; und
R⁵ für F; Cl; Br; C₁- bis C₆-Alkyl; Trifluormethyl; C₁- bis C₆-Alkoxy; Trifluormethoxy; 2,2,2-Trifluorethoxy; C₅- bis C₆-Cycloalkyloxy; Methylthio; Phenoxy, das optional mit R²⁷ substituiert ist, Phenylthio, das optional mit R²⁴ substituiert ist; Benzyloxy, das optional mit R³⁰ auf dem Phenylring substituiert ist, oder ―OC(=O)R²⁸ steht.

19. Zusammensetzung, umfassend eine Verbindung der Formel I nach einem der Ansprüche 13 bis 18 und mindestens eines von (a) einem Tensid, (b) einem organischen Lösungsmittel, und (c) mindestens einem festen oder flüssigen Verdünnungsmittel.

20. Verfahren zum Bekämpfen von Pflanzenkrankheiten, umfassend das Aufbringen einer wirksamen Menge einer fungiziden Zusammensetzung nach einem der Ansprüche 1 bis 8 oder 19 auf die zu schützende Pflanze oder einen Teil von dieser, oder auf den zu schützenden Pflanzensamen oder -sämling.

## Revendications

1. Une composition comprenant
(I) un composé de formule I 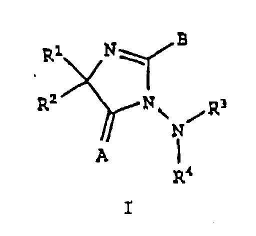 dans laquelle
A est O ou NH ;
B est un halogène ; cyano; R²¹ ; OR²⁹ ; NR⁴⁹ ; R⁶³ ; N=CR⁴⁵R⁴⁶ ; SR⁴⁷ ; ou S(O)₂R⁴⁸ ;
R¹ est un alkyle en C₁-C₄ ; haloalkyle en C₁-C₄ ou vinyle ;
R² est un alkyle en C₂-C₂₀ ; un alkoxyalkyle en C₂-C₂₀ ; un alkyle en C₃-C₈ substitué par un phénoxy ou un phénylthio, chacun éventuellement substitué par R³⁰ ; un cyclonalkyle en C₅-C₇ ; un alkényle en C₂-C₂₀ ; un cycloalkényle en C₅-C₇ ; un phényle éventuellement substitué par R⁵ et R⁷ ; 2-naphtalényle ; un thyényle éventuellement substitué par R⁵ et R⁷ ; un furyle éventuellement substitué par R⁷ ; ou un pyridyle éventuellement substituté par R⁵ et R⁷ ;
avec cette condition que si R² est un phényle et R⁵ est autre que F, R⁵ est fixé en position para par rapport au cycle imidazolinone ;
R³ est un phényle éventuellement substitué par R¹⁰ ;
R⁴ est H ou méthyle ;
R⁵ est un halogène ; nitro ; cyano ; alkyle en C₁-C₆ ; cycloalkyle en C₅-C₆ ; haloalkyle en C₁-C₆ ; alkylthio en C₁-C₆ ; haloalkylthio en C₁-C₆ ; alkoxy en C₁-C₆ ; haloalkoxy en C₁-C₆, cycloalkyloxy en C₅-C₆ ; alkoxyalkyle en C₂-C₆ ; alkoxyalkoxy en C₂-C₆ ; alkényle en C₃-C₆, haloalkényle en C₃-C₆. alkényloxy en C₃-C₆ ; alkynyle en C₃-C₆ ; haloalkynyle en C₃-C₆ ; alkynyloxy en C₃-C₆ ; alkylsulfonyle en C₁-C₆ ; haloalkylsulfonyle en C₁-C₆ ; phényle ou phénylthio, chacun éventuellement substitué par R²⁴ ; phénylméthyle, phénoxyméthyle, phénétyle, ou styryle chacun éventuellement substitué par R²⁴ sur le cycle phényle; phénoxy éventuellement substitué par R²⁷ ; benzyloxy éventuellement substitué par R³⁰ sur le cycle phényle ; -OC(=O)NHR²⁸ ; ou -C(=O)R²⁸ ; ou ―OC(=O)R²⁸
R⁷, R²⁴ et R²⁶ sont indépendamment des 1-2 halogène ; nitro ; alkyle en C₁-C₄ ; trifluorométhyle ; méthylthio ; ou alkoxy en C₁-C₄ ;
R¹⁰ représente des 1-2 substituants choisis dans le groupe consistant en halogène, nitro, cyano, alkyle en C₁-C₄, trifluorométhyle, alkythio en C₁-C₄, alkoxy en C₁-C₄ et trifluorométhoxy ;
R²¹ est un alkyle en C₁-C₄ éventuellement substitué par R⁵¹ ; un alkényle en C₂-C₄, un alkynyle en C₂-C₈, ou un cyclopropyle, chacun éventuellement substitué par 1-3 halogène ; C(=N-V-R⁵³)H ; C(=N-V-R⁵³) (alkyle en C₁-C₄) ; C(=O)OR⁵³ ; C(=O)SR⁵³ ; C(=S)SR⁵³ ; ou C(=O)NR⁵³R⁵⁶ ;
V est O; NR⁵⁵ ; ou une liaison directe ;
R²⁷ est 1-2 halogène ; nitro ; cyano ; alkyle en C₁-C₆ ; haloalkyle en C₁-C₆ ; alkoxy en C₁-C₆ ; haloalkoxy en C₁-C₆ ; alkylsulfonyle en C₁-C₄ ; alkoxyalkyle en C₂-C₆ ; alkylthio en C₁-C₄ ; cycloalkyle en C₅-C₆ ; cycloalkoxy en C₅-C₆ ; alkényle en C₂-C₆ ; haloalkényle en C₂-C₆ ; alkynyle en C₂-C₆ ; hydroxycarbonyle ; alkoxy carbonyle en C₂-C₄ ; ou phénoxy éventuellement substitué par R²⁴ ;
R²⁸ est un alkyle en C₁-C₈ ; ou un phényle ou pyridyle chacun éventuellement substitué par R³⁰ ;
R²⁹ est un alkyle en C₁-C₄ éventuellement substitué par R⁴⁴ ; un alkényle en C₃-C₆, alkynyle en C₃-C₆, ou cyclopropyle chacun éventuellement substitué par 1-3 halogène ; C(=O)R⁵² ; C(=NR⁵⁵)R⁵² ; C(=O)OR⁵³ ; C(=O)NR⁵³R⁵⁶ ; N=CR⁶⁸R⁶⁷ ; ou SO₂R⁵² ;
R³⁰ est 1-2 substituants choisis dans le groupe consistant en halogène, nitro, cyano, alkyl en C₁-C₄, trifluorométhyle, un alkoxy en C₁-C₄ et trifluorométhoxy; ou phénoxy éventuellement substitué par R²⁶ ;
R⁴⁴ est un 1-3 halogène ; un alkoxy en C₁-C₄ ou un haloalkoxy en C₁-C₄ ;
R⁴⁵ est H ou un alkyle en C₁-C₄ ;
R⁴⁶ est H ; un alkyle en C₁-C₄ ; OR⁶⁵ ; ou SR⁶⁵ ;
R⁴⁷ est un alkyle on C₁-C₄ ; un alkényle en C₃-C₄ ; un C(=O)OR⁵² ; ou un C(=O)OR⁵³ ;
R⁴⁸ est un alkyle en C₁-C₄ ;
R⁴⁹ est H ; un alkyle en C₁-C₄ ; un alkényle en C₃-C₄ ; ou un cyclopropyle ;
R⁵¹ est 1-3 halogène ou un haloalkoxy en C₂-C₃ ;
R⁵² et R⁵³ sont chacun indépendamment H ; un alkyle en C₁-C₄ ; ou un alkényle en C₃-C₄ ;
R⁵⁵ et R⁵⁶ sont chacun indépendamment H ou un alkyle en C₁-C₄ ;
R⁶³ un alkyle en C₁-C₄ ;
R⁶³ un alkyle en C₁-C₄ ; ou un alkényle en C₃-C₄ ;
R⁶⁷ est H : ou un alkyle en C₁-C₄ ; et
R⁶⁸ est H ; un alkyle en C₁-C₄ ; ou un haloalkyle en C₁-C₄ ; ou un alkényle en C₂-C₄ ;
à condition que le nombre total de carbones dans R² soit inférieur ou égal à 20; et à condition que si R³ est un phényle ou un cycle hétérocyclique disubstitué par deux groupes alkyle ou alkoxy, ou un alkyle et un groupe alkoxy, un au moins des groupes alkyle et alkoxy est le méthyle ou le méthoxy; et
(2) un ou plusieurs composés choisis parmi monocrotophos, carbofurane, tétrachlovinphos, malathion, parathion-méthyle, méthomyle, chlordimeforme, diazinon, deltaméthrine, oxamyle, fenvalérate, esfenvalérate, permethrine, profenofos, sulprofos, triflumuron, diflubenzuron, méthoprène, buprofézine, thiodicarbe, acéphate, azinphosméthyle, chlorpyrifos, diméthoate, fipronil, flufenprox, fonophos, isofenphos, méthidathion, méthamidophos, phosmet, phosphamidon, phosalone, pirimicarbe, phorate, terbufos, trichlorfon, méthoxychlore, bifenthrine, biphénate, cyfluthrine, fenpropathrine, fluvalinate, flucythrinate, tralomethrine, métaldéhyde, rotenone, carbendazim, thiuram, dodine, manèbe, chloronèbe, benomyle, cymoxanil, fenpropidine, fenpropimorph, triadimefon, captane, thiophanate-méthyle, thiabendazole, phoséthyl-Al, chlorothalonil, dichlorane, métalaxyle, captafol, iprodione, oxadixyle, vinclozoline, kasugamycine, myclobutanil, tebuconazole, difenoconazole, dinicanazole, fluquincanazole, ipconazole, metconazole, penconazole, propiconazole, uniconzole, flutriafol, prochloraze, pyrifenox, fenarimol, triadimenol, diclobutrazol, oxychlorure de cuivre, furalaxyl, folpet, flusilazol, blasticidine S, diclomezine, edifenphos, isoprothiolane, iprobenfos, mepronil, neo-asozine, pencycuron, probenazol, pyroquilon, tricyclazole; validamycine, flutolanil, aldoxycarbe, fenamiphos et fosthietane, oxytetracyline, streptomycine, sulfate de cuivre tribasique, binapacryl, oxythioquinox, chlorobenzilate, dicofol, dienochlore, cyhexatine, hexythiazox, amitraze, propargite, tebufenpyrad, oxyde de fenbutatine, Bacillus thuringiensis, baculovirus et avermectin B.

2. Une composition selon la revendication 1, comprenant en outre au moins un parmi (a) un surfactant, (b) un solvant organique, et (c) au moins un diluant solide ou liquide.

3. Une composition comprenant:
(1) un composé de formule I tel que défini dans la revendication 1 et
(2) au moins un composé insecticide, nématocide, bactéricide, acaricide, composé sémiochimique, repoussant, attirant, phéromone ou stimulant alimentaire.

4. Une composition comprenant:
(1) un compose de formule I selon la revendication 1 ; et
(2) au moins un autre fungicide ayant un mode d'action différent du composé de formule I.

5. Une composition selon une quelconque des revendications 1 à 4 dans laquelle, dans le composé de formule I,
B est halogène, cyano, alkyle en C₁-C₄, ou alkyloxy en C₁-C₄, chacun éventuellement substitué par halogène, alkylthio en C₁-C₄ ; N=CR⁴⁵R⁴⁶ ; NR⁴⁹R⁶³ ; ou S(O)₂(alkyle en C₁-C₄) ;
R¹ est un méthyle ou un halométhyle ;
R² est un alkyle en C₂-C₁₂ ; un alkyle en C₃-C₈ substitué par un phénoxy éventuellement substitué par R³⁰ ; phényle éventuellement substitué par R⁵ et R⁷ ; thiényle éventuellement substitué par R⁷; ou pyridyle éventuellement substitué par R⁵ et R⁷ ;
R³ est un phényle éventuellement substitué par F, Cl, ou méthyle :
R⁴ est H ;
R⁵ est un halogène, nitro, alkyle en C₁-C₆ ; haloalkyle en C₁-C₃ ; méthylthio ; alkoxy en C₁-C₆ ; haloalkoxy en C₁-C₂ ; cycloalkoxy en C₅-C₆ ; phénoxy éventuellement substitué par R²⁷ ; phénylthio substitué par R²⁴ ; phénoxyméthyl éventuellement substitué par R²⁴ sur le cycle phényle; benzyloxy éventuellement substitué par R³⁰ sur le cycle phényle ; ou ―OC=(O)R²⁸ ;
R⁷ et R²⁴ sont indépendamment F; alkyle en C₁-C₂ ; méthylthio ou alkoxy en C₁-C₂ ;
R²⁷ est 1-2 halogène ; cyano ; alkyle en C₁-C₄ ; trifluororméthyle ; alkoxy en C₁-C₄ ; haloalkoxy en C₁-C₄ ; alkylthio en C₁-C₄ ; cycloalkyloxy en C₅-C₆ ; ou allyle ;
R³⁰ est 1-2 halogène ; cyano; alkyle en C₁-C₄ ; trifluorométhyle; alkoxy en C₁-C₄ ; ou trifluorométhoxy ;
R⁴⁶ est H ou un alkyle C₁-C₄ ;
R⁴⁹ et R⁶³ sont chacun indépendamment des alkyles en C₁-C₂.

6. Une composition selon l'une quelconque des revendications 1 à 4 dans laquelle, dans le composé de formule I,
B est F ; Cl ; cyano ; alkyle en C₁-C₂ ; alkylthio en C₁-C₂ ; alkoxy en C₁-C₂ ; N=CR⁴⁵R⁴⁶ ; NMe₂ ; ou S(O)₂(alkyle en C₁-C₂) ;
R¹ est méthyle ;
R² est un alkyle en C₁₂-C₁₂ ; phényle éventuellement substitué par R⁵ et R⁷ ; ou thiényle éventuellement substitué par R⁵ et R⁷ ; et
R⁵ est F ; Cl ; Br ; alkyle en C₁-C₆ ; trifluorométhyle ; alkoxy en C₁-C₆ ; trifluorométhoxy ; 2,2,2-trifiuoroéthoxy ; cycloalkyloxy en C₅-C₆ ; méthylthio ; phénoxy éventuellement substitué par R²⁷ ; phénylthio éventuellement substitué par R²⁴ ; benzyloxy eventuellement substitué par R³⁰ sur le cycle phényle ; ou ―OC(=O)R²⁸.

7. Une composition selon une quelconque des revendications 1 à 6, dans laquelle R¹ et R² sont différents dans le composé de formule I, et le composé de formule I est sous une forme énantiomériquement pure.

8. Une composition selon une quelconque des revendications 1 à 7, dans laquelle le composé de formule I est la 3,5-dihydro-5 -méthyl-2-(méthylthio)-5-phényl-3-(phénylamino)-4*H*-imidazol-4-one.

9. Une méthode pour combattre les maladies des plantes provoquées par des champignons choisis parmi le groupe constitué par *Peronospora tabacina*, *Pseudoperonospora cubensis*. *Pythium aphanidermatum*, *Alternaria brassicae*, *Cercosporidium personatum*, *Cercospora arachidicola*, *Cercospora beticola*, *Botrytis cinerea*, *Monolinia fructicola*, *Pyricularia oryzae*, *Podosphaera leucotricha*, *Venturia inaequalis*, *Erysiphe graminis*, *Uncinula necatur*, *Puccinia graminis*, *Hemileia vastatrix*, *Puccinia striiformis*, *Puccinia arachidis*, *Sphaerotheca fuliginea*, *Verticillium dahliae*, *Pythium aphanidermatum*, et *Phylophthora megasperma*, comprenant l'application sur la plante ou une de ses parties à protéger d'une quantité efficace d'une composition fongicide comprenant un composé de formule I selon la revendication 1.

10. Une méthode selon la revendication 9, dans laquelle les champignons choisis parmi le groupe de *Erysiphe graminis*, *Pyricularia oryzae* et *Botrytis cinerea* sont contrôlés.

11. Une méthode selon une quelconque des revendications 9 ou 10 dans laquelle, dans les composés de formule I, R¹ et R² sont différents et le composé est sous une forme énantiomériquement pure.

12. Une méthode selon une quelconque des revendications 9 à 11, dans laquelle le composé de formule I est la 3,5-dihydro-5-méthyl-2-(méthylthio)-5-phényl-3-(phénylamino}-4*H*-imidazol-4-one.

13. Un composé de formule I tel que défini dans la revendication 1, 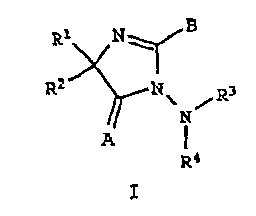 avec cette condition que si A représente O, et soit (a) B désigne OR²⁹ et R²⁹ désigne un alkyle en C₁-C₄ éventuellement substitué par R⁴⁴, un alkényle en C₃-C₆, ou un alkynyle en C₃-C₆, ou bien (b) B désigne SR⁴⁷ et R⁴⁷ désigne un alkyle en C₁-C₄ ou un alkényle en C₃-C₄,
alors R¹ et R² sont différents, et le composé n'est pas sous forme racémique.

14. Un composé selon la revendication 13, dans lequel R¹ et R² sont différents, qui présente la stéréochimie

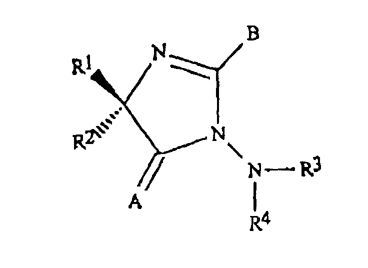

15. Un composé selon la revendication 13 ou la revendication 14, qui est 3,5-dihydro-5-méthyl-2-(méthylthio)-5-phényl-3-(phénylamino)-4-*H*-imidazol-4-one.

16. Un composé de formule I selon la revendication 1, avec cette condition que si A désigne O et soit (a) B désigne OR²⁹, alors R²⁹ ne signifie pas un alkyle en C₁-C₄ éventuellement substitué par R⁴⁴, ni un alkényle on C₃-C₆ ni un alkynyle en C₃-C₆, soit (b) B désigne SR⁴⁷, alors R⁴⁷ ne désigne pas un alkyle en C₁-C₄ ni un alkényle en C₃-C₄.

17. Un composé selon la revendication 16, dans lequel
B est un halogène; cyano; alkyle en C₁-C₄, ou alkoxy en C₁-C₄ chacun éventuellement substitué par un halogène; un alkylthio en C₁-C₄ ; N=CR⁴⁵R⁴⁶ ; NR⁴⁹R⁶³ ; ou S(O)₂ (alkyle en C₁-C₄) ;
R¹ est un méthyle ou un halométhyle ;
R² est un alkyle en C₂-C₁₂ ; alkyle en C₃-C₈ substitué par un groupe phénoxy éventuellement substitué par R³⁰ ; phényle éventuellement substitué par R⁵ et R⁷ ; thiényle éventuellement substitué par R⁷ ; ou pyridyle éventuellement substitué par R⁵ et R⁷ ;
R³ est un phényle éventuellement substitué par F ; Cl ; ou méthyle
R⁴ est H ;
R⁵ est un halogène ; nitro ; un alkyle en C₁-C₆ ; haloalkyle en C₁-C₃ ; méthylthio ; alkoxy on C₁-C₆ ; haloalkoxy en C₁-C₂ ; cycloalkyloxy en C₅-C₆ ; phénoxy éventuellement substitué par R²⁷ ; phénylthio substitué par R²⁴ ; phénoxyméthyle substitué éventuellement par R²⁴ sur le cycle phényle; benzyloxy éventuellement substitué par R³⁰ sur le cycle phényle ; ou -OC(=O)R²⁸ ;
R⁷ et R²⁴ sont indépendamment F ; alkyle on C₁-C₂ ; méthylthio ; ou alkoxy en C₁-C₂ ;
R²⁷ est un 1,2-halogène ; un cyano ; un alkyle en C₁-C₄ ; trifluorométhyle ; alkoxy en C₁-C₄ ; haloalkoxy en C₁-C₄ ; alkylthio on C₁-C₄ ; cycloalkoxy en C₅-C₆ ; ou allyle ;
R³⁰ est 1,2-halogène ; cyano ; alkyle en C₁-C₄ ; trifluorométhyle ; alkoxy en C₁-C₄, ou trifluorométhyle ;
R⁴⁶ est H ou un alkyle on C₁-C₄ ;
R⁴⁹ et R⁶³ sont chacun indépendamment des alkyles C₁-C₂.

18. Un composé selon la revendication 16, dans lequel
B est F ; Cl ; cyano ; alkyle en C₁-C₂ ; alkylthio en C₁-C₂ ; N=CR⁴⁵R⁴⁶ ; NMe₂ ; ou S(O)₂(alkyle en C₁-C₂) ;
R¹ est un méthyle ;
R² est un alkyle en C₂-C₁₂ ; phényle éventuellement substitué par R⁵ et R⁷ ; ou thiényle éventuellement substitué par R⁵ et R⁷ ; et
R⁵ est F ; Cl ; Br ; alkyle en C₁-C₆ ; trifluorométhyle ; alkoxy en C₁-C₆ ; trifluorométhoxy; 2,2,5-trifluoroéthoxy ; cycloalkyloxy en C₅-C₆ ; méthylthio ; phénoxy éventuellement substitué par R²⁷ ; phénylthio éventuellement substitué par R²⁴ ; benzyloxy éventuellement substitué par R³⁰ sur le cycle phényle ; ou ―OC(=O)R²⁸.

19. Une composition comprenant un composé de formule I, selon une quelconque des revendications 13 à 18, et au moins un choisi parmi (a) un agent tensioactif; (b) un solvant organique; et (c) au moins diluant solide ou liquide

20. Une méthode pour combattre les maladies des plantes comprenant l'application à la plante ou à une partie de la plante à protéger, ou à la graine de la plante ou aux pousses à protéger, d'une quantité efficace d'une composition fongicide selon une quelconque des revendications 1 à 8, ou 19.
